# EUROPEAN PATENT SPECIFICATION

(11) **EP 4 346 581 B1**
(45) Date of publication and mention of the grant of the patent: **21.10.2026**
(21) Application number: 22813879.8
(22) Date of filing: 02.06.2022
(51) Int. Cl.: G16H 40/67, G16H 50/30, F02D 41/14, A01B 59/00, G16H 20/30, G16H 20/60, A61B 5/097, G16H 20/70

(54) **A SYSTEM AND METHOD FOR ESTIMATING GREENHOUSE GAS EMISSIONS IN AN ENVIRONMENT HOUSING AN ANIMAL POPULATION**
SYSTEM UND VERFAHREN ZUR SCHÄTZUNG VON TREIBHAUSGASEMISSIONEN IN EINER UMGEBUNG MIT EINER TIERPOPULATION
SYSTÈME ET PROCÉDÉ D'ESTIMATION D'ÉMISSIONS DE GAZ À EFFET DE SERRE DANS UN ENVIRONNEMENT HÉBERGEANT UNE POPULATION D'ANIMAUX

(30) Priority: 03.06.2021 ES 202130513; 06.06.2021 IL 28374121
(43) Date of publication of application: 10.04.2024
(73) Proprietor: S.C.R. (Engineers) Limited, 4250553 Netanya (IL); Merck Sharp & Dohme Animal Health, S.L., 37008 Carbajosa de la Sagrada Salamanca (ES)
(72) Inventor: GENZEL, Eran, 4250553 Netanya (IL); RABINOVITZ, Rotem, 4250553 Netanya (IL); BAR, Doron, 4250553 Netanya (IL); CAMPILLO BENÉITEZ, Juan Pedro, 37008 Carbajosa de la Sagrada Salamanca (ES)
(74) Representative: Marks & Clerk LLP
(86) International application number: PCT/IL2022/050586
(87) International publication number: WO 2022/254441

(56) References cited:
- WO-A1-2017/122097
- WO-A1-2017/122097
- US-A1- 2016 324 188
- US-A1- 2020 273 047
- US-A1- 2021 148 891
- OPIO C., GERBER, P., MOTTET, A., FALCUCCI, A., TEMPIO, G., MACLEOD, M., VELLINGA, T., HENDERSON, B. & STEINFELD, H.: "Greenhouse gas emissions from ruminant supply chains", A GLOBAL LIFE CYCLE ASSESSMENT, 1 January 2013 (2013-01-01), pages 1 - 214, XP093010534

## Description

### TECHNICAL FIELD

The presently disclosed subject matter relates to greenhouse gas emission estimation and reduction, and more specifically to greenhouse gas emission estimation and reduction in an environment housing an animal population.

### BACKGROUND

Greenhouse gas (also referred to as GHG) absorbs Infra-Red (IR) radiation emitted from earth's surface, and redirects it back to earth's surface, causing global warming. Livestock are responsible for a considerable amount of global greenhouse gases, evaluated by some at 14.5%. In view of the significance of livestock contribution to global greenhouse gas emissions, many studies have been made, showing that improving fertility, health, feed and herd management can have a substantial contribution to reducing livestock greenhouse gas emissions, and improving the ratio of GHG emission to milk production (so as to generate less GHG per milk unit generated by a given animal population).

Nowadays, greenhouse gas emission in environments housing animal populations is mainly determined by a subjective human auditor estimating the greenhouse gas emissions, or by direct measurements acquired by dedicated GHG measurement instrumentation.

Human auditors base their GHG emission estimation on data collected in a slow and manual process that is prone to errors. As part of the GHG emission estimation process, the human auditors visit and visually inspect the facilities housing the animals, and gather various types of data on the animal population itself. In some cases, the data required for the human auditor to be able to estimate GHG emissions, or parts thereof, is not available (at times due to the fact that it is not collected). Even when such data is available, it is non-homogenous as different facility owners collect different data, using different data collection methods. Based at least in part on the human auditor's subjective impression, and based on the non-homogenous data (which may also be partial), the human auditor estimates the GHG emissions in the audited environment.

One exemplary problem of the subjectivity of an auditor is its first impression of an animal facility. For a human auditor, it may be hard to shake off a first impression of a facility. Such first impression can lead to erroneous assessment of GHG emissions in such facility. For example, if the animal facility makes a good first impression, the auditor may miss out on other problems, or give them lesser weight, or vice versa.

It can be appreciated that efforts are made to train auditors to maintain standard levels of evaluation, however interobserver reliability and consistency can be low despite training of the human auditors. Even in those cases where auditors undergo identical training, that training needs to be continuous to hold high standards of consistency, and even in these cases, the subjectiveness bias is not, and cannot, be completely irradicated.

Thus, current GHG emission estimation methods are prone to the subjectivity of the auditor. On top of that, they are also sensitive to the data collection method, sensitive to the timing, circumstances, and frequency of the inspection (lighting conditions, weather conditions, etc.), and require long gathering and processing times.

In addition, GHG emission estimations may vary between auditors, and between audits, because they are not produced in real-time, nor are they based on continuous monitoring. Human auditing is not based on continuous, or near continuous, monitoring of various parameters (e.g., parameters relating to the animals' environment, the animals' health measures, animal treatments, the weather, etc.), inter alia because it is impossible for a human to track such amounts of data, let alone track such amounts of data in a manner that will enable evaluating GHG emissions in the required speed. To the contrary, having an automatic mechanism for estimating GHG emissions, optionally in real-time or near real-time, continuously or near continuously, can enable a much more complete and more accurate GHG emission estimation that is not dependent on outdated or no-longer relevant data.

As mentioned above, GHG emissions can be measured directly using dedicated instrumentation (e.g. Respiratory Chamber, Tracer technique (SF₆), Non dispersive Infrared Methane detector, Micro - Meteorological Techniques, Laser Methane Detector (LMD), etc.), however such instrumentation is usually unavailable for livestock growers to continuously and directly measure greenhouse gas emissions in the environment in which their livestock is grown. Direct measurement of GHG emissions is also an extremely difficult task to perform when the animals are not within a closed housing in which the dedicated instrumentation can yield accurate results. For example, dedicated instrumentation cannot provide an accurate measurement when the animal population is grazing in open pasture.

In spite of the critical effect of livestock on global greenhouse gas emission, existing solutions do not offer non-biased, continuous, or near continuous, visibility to the stakeholders of this industry as to GHG emission in the environment in which the livestock is grown.

Thus, there is a real need for a new and improved system and a method for estimating greenhouse gas emissions in an environment housing an animal population.

Document US 2021/148891 Al discloses lifecycle assessment systems and methods for determining emissions from animal production.

### Summary of the invention

The invention is set out in the appended claims.

### Summary of related disclosure

In accordance with a first aspect of the presently disclosed subject matter, there is provided a system for estimating greenhouse gas emission in an environment housing an animal population, the system comprising: one or more monitoring devices configured to monitor parameters of members of the animal population; a data repository comprising one or more records, each of the records (i) being associated with a respective member of the members, and (ii) including one or more parameters of the respective member as monitored by at least one of the monitoring devices over time; and a processing circuitry configured to: obtain at least a subset of the records, the subset being associated with a group of members of the animal population; determine one or more greenhouse gas emission affecting parameters based on the subset; estimate an amount of greenhouse gas emission in the environment utilizing the greenhouse gas emission affecting parameters; and perform an action utilizing the estimated amount of greenhouse gas emission in the environment.

In one embodiment of the presently disclosed subject matter and/or embodiments thereof, the estimation is based on comparison of the greenhouse gas emission affecting parameters to a given baseline.

In one embodiment of the presently disclosed subject matter and/or embodiments thereof, the baseline is geographical location specific.

In one embodiment of the presently disclosed subject matter and/or embodiments thereof, the baseline is determined based on historical values of the greenhouse gas emission affecting parameters.

In one embodiment of the presently disclosed subject matter and/or embodiments thereof, the baseline is determined using greenhouse gas emission measurements acquired using greenhouse gas emission measuring equipment.

In one embodiment of the presently disclosed subject matter and/or embodiments thereof, the greenhouse gas emission measurements are acquired from the environment.

In one embodiment of the presently disclosed subject matter and/or embodiments thereof, the action is one or more of: (a) providing a user of the system with an indication of the estimated amount of greenhouse gas emission, or (b) suggesting one or more greenhouse gas emission reduction actions to be taken on the animal population in order to reduce the greenhouse gas emission in the environment.

In one embodiment of the presently disclosed subject matter and/or embodiments thereof, the greenhouse gas emission reduction actions include one or more of: administering a treatment to the animal population, changing a temperature of an environment of the animal population, changing a feed of the animal population, or changing a schedule of the animal population.

In one embodiment of the presently disclosed subject matter and/or embodiments thereof, the greenhouse gas emission affecting parameters include one or more of the following: (a) a welfare score indicative of a health state of the group of members, (b) Heat Detection Rate (HDR) calculated for the group of members, (c) a conception rate calculated for the group of members, (d) a health score calculated for the group of members, (e) rumination consistency calculated for the group of members, or (f) rumination time heterogeneity calculated for the group of members.

In one embodiment of the presently disclosed subject matter and/or embodiments thereof, the processing circuitry is further configured to calculate, based on the subset of records, for each given member of the group of members, at least one of: (a) an animal health score indicative of a health state of the respective member, (b) an animal natural living score, indicative of compliance of a behavior pattern of the respective member with a desired natural behavior pattern, or (c) an animal affectivity/happiness score, indicative of compliance of an affectivity/happiness measure of the respective member with a desired affectivity/happiness measure; and wherein: the health score is calculated based on the animal health scores calculated for the group of members; and the welfare score is calculated based on the at least one of: (a) the health score, (b) the animal natural living scores calculated for the group of members, or (c) the animal affectivity/happiness scores calculated for the group of members.

In one embodiment of the presently disclosed subject matter and/or embodiments thereof, the parameters include one or more of the following behavioral parameters: (a) percentage of movement time within a first time period of the given member, (b) percentage of feeding time within a second time period of the given member or (c) percentage of social behavior time within a third time period of the given member; and wherein the animal natural living score for the given member is determined based on the behavioral parameters.

In one embodiment of the presently disclosed subject matter and/or embodiments thereof, the natural living score for the given member is determined based on consistency of values of at least some of the parameters over time.

In one embodiment of the presently disclosed subject matter and/or embodiments thereof, the consistency of the values is measured against reference parameters.

In one embodiment of the presently disclosed subject matter and/or embodiments thereof, the reference parameters are measured from reference animals.

In one embodiment of the presently disclosed subject matter and/or embodiments thereof, the parameters include one or more of the following affectivity/happiness parameters: (a) respiration level of the given member, (b) percentage of rumination time within a fourth time period of the given member, or (c) percentage of feeding time within a fifth time period of the given member; and wherein the affectivity/happiness score for the given member is determined based on the affectivity/happiness parameters.

In one embodiment of the presently disclosed subject matter and/or embodiments thereof, the affectivity/happiness score for the given member is determined based on consistency of values of at least some of the parameters over time.

In one embodiment of the presently disclosed subject matter and/or embodiments thereof, the consistency of the values is measured against reference parameters.

In one embodiment of the presently disclosed subject matter and/or embodiments thereof, the reference parameters are measured from reference animals.

In one embodiment of the presently disclosed subject matter and/or embodiments thereof, the welfare score is calculated based on a variation between the affectivity/happiness scores of the group of the members.

In one embodiment of the presently disclosed subject matter and/or embodiments thereof, the records also include one or more environmental parameters, indicative of the state of the environment of the respective member and wherein the determination of (a) the animal natural living score of the respective member or (b) of the animal affectivity/happiness score of the respective member, is also based on the environmental parameters.

In one embodiment of the presently disclosed subject matter and/or embodiments thereof, the monitoring device includes one or more of: an accelerometer, a temperature sensor, a location sensor, a pedometer, or a heart rate sensor.

In one embodiment of the presently disclosed subject matter and/or embodiments thereof, at least some of the records further include descriptive data associated with the respective member, wherein the descriptive data is not obtained from the monitoring device.

In one embodiment of the presently disclosed subject matter and/or embodiments thereof, the descriptive data includes one or more of: age of the respective member, sex of the respective member, treatment history of the respective member, or genetic information associated with the respective member.

In one embodiment of the presently disclosed subject matter and/or embodiments thereof, the at least subset of the records are all the records of the animal population.

In one embodiment of the presently disclosed subject matter and/or embodiments thereof, the animal population is located in one or more geographical sites.

In one embodiment of the presently disclosed subject matter and/or embodiments thereof, at least some of the monitoring devices are attached monitoring devices, attached to respective members.

In one embodiment of the presently disclosed subject matter and/or embodiments thereof, the attached monitoring devices are monitoring tags or monitoring collars.

In one embodiment of the presently disclosed subject matter and/or embodiments thereof, the animal population is a population of ruminating animals or companion animals.

In accordance with a second aspect of the presently disclosed subject matter, there is provided a method for estimating greenhouse gas emission in an environment housing an animal population, the system comprising: obtaining, by a processing circuitry, at least a subset a group of one or more records, each of the records (i) being associated with a respective member of members of the animal population, and (ii) including one or more parameters of the respective member as monitored over time by at least one of a group of one or more monitoring devices configured to monitor parameters of the members of the animal population, wherein the subset is associated with a first group of members of the animal population; determining, by the processing circuitry, one or more greenhouse gas emission affecting parameters based on the subset; estimating, by the processing circuitry, an amount of greenhouse gas emission in the environment utilizing the greenhouse gas emission affecting parameters; and performing, by the processing circuitry, an action utilizing the estimated amount of greenhouse gas emission in the environment.

In one embodiment of the presently disclosed subject matter and/or embodiments thereof, the estimating is based on comparison of the greenhouse gas emission affecting parameters to a given baseline.

In one embodiment of the presently disclosed subject matter and/or embodiments thereof, the baseline is geographical location specific.

In one embodiment of the presently disclosed subject matter and/or embodiments thereof, the baseline is determined based on historical values of the greenhouse gas emission affecting parameters.

In one embodiment of the presently disclosed subject matter and/or embodiments thereof, the baseline is determined using greenhouse gas emission measurements acquired using greenhouse gas emission measuring equipment.

In one embodiment of the presently disclosed subject matter and/or embodiments thereof, the greenhouse gas emission measurements are acquired from the environment.

In one embodiment of the presently disclosed subject matter and/or embodiments thereof, the action is one or more of: (a) providing a user of the system with an indication of the estimated amount of greenhouse gas emission, or (b) suggesting one or more greenhouse gas emission reduction actions to be taken on the animal population in order to reduce the greenhouse gas emission in the environment.

In one embodiment of the presently disclosed subject matter and/or embodiments thereof, the greenhouse gas emission reduction actions include one or more of: administering a treatment to the animal population, changing a temperature of an environment of the animal population, changing a feed of the animal population, or changing a schedule of the animal population.

In one embodiment of the presently disclosed subject matter and/or embodiments thereof, the greenhouse gas emission affecting parameters include one or more of the following: (a) a welfare score indicative of a health state of the group of members, (b) Heat Detection Rate (HDR) calculated for the group of members, (c) a conception rate calculated for the group of members, (d) a health score calculated for the group of members, (e) rumination consistency calculated for the group of members, or (f) rumination time heterogeneity calculated for the group of members.

In one embodiment of the presently disclosed subject matter and/or embodiments thereof, the method further comprises calculating, based on the subset of records, for each given member of the group of members, at least one of: (a) an animal health score indicative of a health state of the respective member, (b) an animal natural living score, indicative of compliance of a behavior pattern of the respective member with a desired natural behavior pattern, or (c) an animal affectivity/happiness score, indicative of compliance of an affectivity/happiness measure of the respective member with a desired affectivity/happiness measure; and wherein: the health score is calculated based on the animal health scores calculated for the group of members; and the welfare score is calculated based on the at least one of: (a) the health score, (b) the animal natural living scores calculated for the group of members, or (c) the animal affectivity/happiness scores calculated for the group of members.

In one embodiment of the presently disclosed subject matter and/or embodiments thereof, the parameters include one or more of the following behavioral parameters: (a) percentage of movement time within a first time period of the given member, (b) percentage of feeding time within a second time period of the given member or (c) percentage of social behavior time within a third time period of the given member; and wherein the animal natural living score for the given member is determined based on the behavioral parameters.

In one embodiment of the presently disclosed subject matter and/or embodiments thereof, the natural living score for the given member is determined based on consistency of values of at least some of the parameters over time.

In one embodiment of the presently disclosed subject matter and/or embodiments thereof, the consistency of the values is measured against reference parameters.

In one embodiment of the presently disclosed subject matter and/or embodiments thereof, the reference parameters are measured from reference animals.

In one embodiment of the presently disclosed subject matter and/or embodiments thereof, the parameters include one or more of the following affectivity/happiness parameters: (a) respiration level of the given member, (b) percentage of rumination time within a fourth time period of the given member, or (c) percentage of feeding time within a fifth time period of the given member; and wherein the affectivity/happiness score for the given member is determined based on the affectivity/happiness parameters.

In one embodiment of the presently disclosed subject matter and/or embodiments thereof, the affectivity/happiness score for the given member is determined based on consistency of values of at least some of the parameters over time.

In one embodiment of the presently disclosed subject matter and/or embodiments thereof, the consistency of the values is measured against reference parameters.

In one embodiment of the presently disclosed subject matter and/or embodiments thereof, the reference parameters are measured from reference animals.

In one embodiment of the presently disclosed subject matter and/or embodiments thereof, the welfare score is calculated based on a variation between the affectivity/happiness scores of the group of the members.

In one embodiment of the presently disclosed subject matter and/or embodiments thereof, the records also include one or more environmental parameters, indicative of the state of the environment of the respective member and wherein the determination of (a) the animal natural living score of the respective member or (b) of the animal affectivity/happiness score of the respective member, is also based on the environmental parameters.

In one embodiment of the presently disclosed subject matter and/or embodiments thereof, the monitoring device includes one or more of: an accelerometer, a temperature sensor, a location sensor, a pedometer, or a heart rate sensor.

In one embodiment of the presently disclosed subject matter and/or embodiments thereof, at least some of the records further include descriptive data associated with the respective member, wherein the descriptive data is not obtained from the monitoring device.

In one embodiment of the presently disclosed subject matter and/or embodiments thereof, the descriptive data includes one or more of: age of the respective member, sex of the respective member, treatment history of the respective member, or genetic information associated with the respective member.

In one embodiment of the presently disclosed subject matter and/or embodiments thereof, the at least subset of the records are all the records of the animal population.

In one embodiment of the presently disclosed subject matter and/or embodiments thereof, the animal population is located in one or more geographical sites.

In one embodiment of the presently disclosed subject matter and/or embodiments thereof, at least some of the monitoring devices are attached monitoring devices, attached to respective members.

In one embodiment of the presently disclosed subject matter and/or embodiments thereof, the attached monitoring devices are monitoring tags or monitoring collars.

In one embodiment of the presently disclosed subject matter and/or embodiments thereof, the animal population is a population of ruminating animals or companion animals.

In accordance with a second aspect of the presently disclosed subject matter, there is provided a non-transitory computer readable storage medium having computer readable program code embodied therewith, the computer readable program code, executable by at least one processing circuitry of a computer to perform a method for estimating greenhouse gas emission in an environment housing an animal population, the method comprising: obtaining, by a processing circuitry, at least a subset a group of one or more records, each of the records (i) being associated with a respective member of members of the animal population, and (ii) including one or more parameters of the respective member as monitored over time by at least one of a group of one or more monitoring devices configured to monitor parameters of the members of the animal population, wherein the subset is associated with a first group of members of the animal population; determining, by the processing circuitry, one or more greenhouse gas emission affecting parameters based on the subset; estimating, by the processing circuitry, an amount of greenhouse gas emission in the environment utilizing the greenhouse gas emission affecting parameters; and performing, by the processing circuitry, an action utilizing the estimated amount of greenhouse gas emission in the environment.

### BRIEF DESCRIPTION OF THE DRAWINGS

In order to understand the presently disclosed subject matter and to see how it may be carried out in practice, the subject matter will now be described, by way of non-limiting examples only, with reference to the accompanying drawings, in which:
**Fig. 1** is a schematic illustration of components of a welfare score for an animal population, in accordance with the presently disclosed subject matter;
**Fig. 2** is a block diagram schematically illustrating one example of a welfare determination system for determining the welfare of an animal population, in accordance with the presently disclosed subject matter;
**Fig. 3** is a flowchart illustrating one example of a sequence of operations carried out for determining the welfare of an animal population, in accordance with the presently disclosed subject matter;
**Fig. 4** is a schematic illustration of the determination of welfare KPI scores for an animal population, in accordance with the presently disclosed subject matter;
**Fig. 5A** is an illustration of one example of a Graphical User Interface (GUI) for a welfare determination system, in accordance with the presently disclosed subject matter;
**Fig. 5B** is an illustration of another example of a GUI for a welfare determination system, in accordance with the presently disclosed subject matter;
**Fig. 6** is a schematic illustration of the determination of welfare thresholds in various countries, in accordance with the presently disclosed subject matter;
**Fig. 7** is a schematic illustration of the effects of weather and seasons on welfare scores, in accordance with the presently disclosed subject matter;
**Fig. 8** is a schematic illustration of an exemplary comparison of human auditors determined welfare scores and welfare KPI scores determined by the system in various facilities, in accordance with the presently disclosed subject matter;
**Fig. 9** is a schematic illustration of an example of the consistency between human auditors determined welfare scores and welfare KPI scores determined by the system, in accordance with the presently disclosed subject matter;
**Fig. 10** is a block diagram schematically illustrating one example of a greenhouse gas emission estimation and reduction system, in accordance with the presently disclosed subject matter;
**Fig. 11** is a flowchart illustrating one example of a sequence of operations carried out for estimating an amount of greenhouse gas emission in an environment housing an animal population, in accordance with the presently disclosed subject matter;
**Fig 12** is a schematic illustration of a correlation between heat detection rates/ conception rates and methane output, in accordance with the presently disclosed subject matter; and
**Fig 13** is a schematic illustration of a relationship between yield of methane and carbon dioxide per units of milk.

### DETAILED DESCRIPTION

In the following detailed description, numerous specific details are set forth in order to provide a thorough understanding of the presently disclosed subject matter. However, it will be understood by those skilled in the art that the presently disclosed subject matter may be practiced without these specific details. In other instances, well-known methods, procedures, and components have not been described in detail so as not to obscure the presently disclosed subject matter.

In the drawings and descriptions set forth, identical reference numerals indicate those components that are common to different embodiments or configurations.

Unless specifically stated otherwise, as apparent from the following discussions, it is appreciated that throughout the specification discussions utilizing terms such as "obtaining", "determining", "estimating", "performing", "suggesting", "inserting", "analyzing", "providing", "calculating", "suggesting" or the like, include action and/or processes of a computer that manipulate and/or transform data into other data, said data represented as physical quantities, e.g. such as electronic quantities, and/or said data representing the physical objects. The terms "computer", "processor", "processing circuitry" and "controller" should be expansively construed to cover any kind of electronic device with data processing capabilities, including, by way of non-limiting example, a personal desktop/laptop computer, a server, a computing system, a communication device, a smartphone, a tablet computer, a smart television, a processor (e.g. digital signal processor (DSP), a microcontroller, a field programmable gate array (FPGA), an application specific integrated circuit (ASIC), etc.), a group of multiple physical machines sharing performance of various tasks, virtual servers co-residing on a single physical machine, any other electronic computing device, and/or any combination thereof.

The operations in accordance with the teachings herein may be performed by a computer specially constructed for the desired purposes or by a general-purpose computer specially configured for the desired purpose by a computer program stored in a non-transitory computer readable storage medium. The term "non-transitory" is used herein to exclude transitory, propagating signals, but to otherwise include any volatile or nonvolatile computer memory technology suitable to the application.

As used herein, the phrase "for example," "such as", "for instance" and variants thereof describe non-limiting embodiments of the presently disclosed subject matter. Reference in the specification to "one case", "some cases", "other cases" or variants thereof means that a particular feature, structure or characteristic described in connection with the embodiment(s) is included in at least one embodiment of the presently disclosed subject matter. Thus, the appearance of the phrase "one case", "some cases", "other cases" or variants thereof does not necessarily refer to the same embodiment(s).

It is appreciated that, unless specifically stated otherwise, certain features of the presently disclosed subject matter, which are, for clarity, described in the context of separate embodiments, may also be provided in combination in a single embodiment. Conversely, various features of the presently disclosed subject matter, which are, for brevity, described in the context of a single embodiment, may also be provided separately or in any suitable sub-combination.

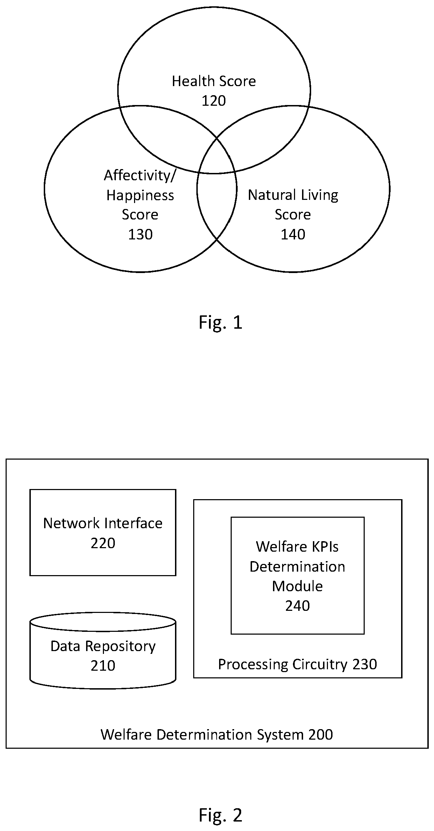

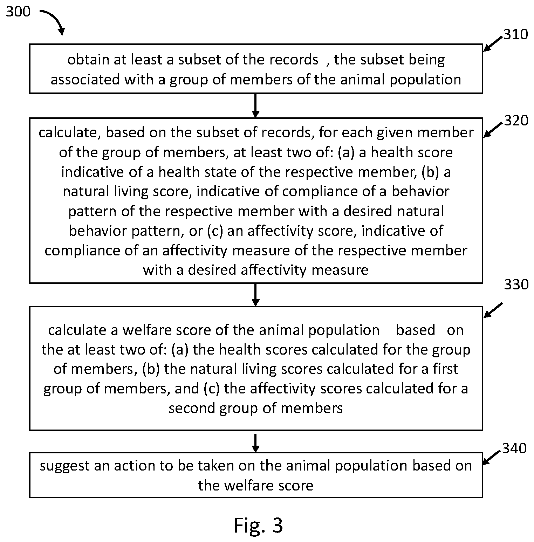

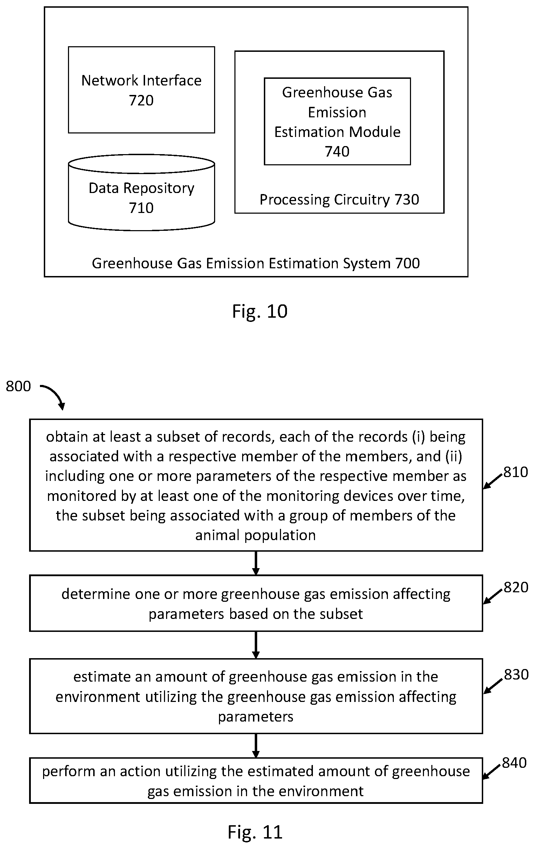

In embodiments of the presently disclosed subject matter, fewer, more and/or different stages than those shown in **Figs. 3** **and** **11** may be executed. In embodiments of the presently disclosed subject matter one or more groups of stages illustrated in **Figs. 3** **and** **11** may be executed simultaneously. **Figs. 2** **and** **10** illustrates a general schematic of the system architecture in accordance with an embodiment of the presently disclosed subject matter. Each module in **Figs. 2** **and** **10** can be made up of any combination of software, hardware and/or firmware that performs the functions as defined and explained herein. The modules in **Figs. 2** **and** **10** may be centralized in one location or dispersed over more than one location, as detailed herein. In other embodiments of the presently disclosed subject matter, the system may comprise fewer, more, and/or different modules than those shown in **Figs. 2** **and** **10****.**

Any reference in the specification to a method should be applied mutatis mutandis to a system capable of executing the method and should be applied mutatis mutandis to a non-transitory computer readable medium that stores instructions that once executed by a computer result in the execution of the method.

Any reference in the specification to a system should be applied mutatis mutandis to a method that may be executed by the system and should be applied mutatis mutandis to a non-transitory computer readable medium that stores instructions that may be executed by the system.

Any reference in the specification to a non-transitory computer readable medium should be applied mutatis mutandis to a system capable of executing the instructions stored in the non-transitory computer readable medium and should be applied mutatis mutandis to method that may be executed by a computer that reads the instructions stored in the non-transitory computer readable medium.

Bearing this in mind, attention is drawn to **Fig. 1****,** which is a schematic illustration of components of a welfare score for an animal population, in accordance with the presently disclosed subject matter.

Before turning to describe the figure, it is to be noted that the terms affectivity and happiness are used herein interchangeably with respect to the positive or negative effects of the environment on an animal population or part thereof and/or with respect to indications on the mental and/or emotional states of the animal population or part thereof.

Turning to the figure, in accordance with the schematic illustration shown therein, a welfare score for an animal population (or a part thereof) is determined based on at least one of the following welfare Key Performance Indicators (KPIs): a health score 120, an affectivity/happiness score 130 and a natural living score 140.

The calculation of these welfare KPIs (the health score 120, the affectivity/happiness score 130 and the natural living score 140) is based on data associated with members of the animal population. The data used for calculation of the welfare KPIs can be descriptive data (e.g., ages of the members, sex of the members, treatment history of the members, genetic information associated with the members, etc.) and/or monitoring data (e.g., parameters of the member of the animal population that are monitored over time using one or more monitoring devices, at least part of which can optionally be attached to the member), and/or data originating from other resources, as further detailed herein.

The monitoring data relating to each member of the animal population can include one or more of: location information (indicative of the geographical locations of the member), body temperature, respiration type, respiration levels, rumination time, movement type, movement time, feeding time, socialism time, elimination behaviors, internal rumen environment parameters (which can be monitored, for example, using a rumen bolus), etc.

The monitoring data can be obtained from sensors comprised within the monitoring devices, such as: one or more accelerometers, a temperature sensor, a location sensor (e.g., a Global Positioning System (GPS) receiver comprised within the monitoring device attached to the member), a thermal sensor, a pedometer, an animal identification component (e.g. an Identification (ID) Tag), a heart rate sensor, a biosensor, or any other sensor that can be used to monitor one or more parameters of the member of the animal population.

It is to be noted that in some cases, at least parts of the monitoring data can be measured over time. For example, rumination time, movement time, feeding time, socialism time, etc., can be the time from start to end of the respective activity. For example, if a member of the animal population started eating at 10:00 and finished eating at 10:30, the feeding time is 30 minutes. It is to be noted further that in some cases, at least parts of the monitoring data can be measured periodically, near continuously, or continuously, or in some combination (where some data is collected periodically and some data is collected continuously or near continuously). For example, body temperature, location, heart rate, etc. can be measured on a periodical basis (e.g., every minute, every 10 minutes, etc.), or continuously.

It is to be noted that whenever reference is made to continuously, near-continuously, real-time or near real time, the time intervals for obtaining a measurement can be milliseconds, seconds, minutes, hours or days, while noting that in comparison to current solutions in which human auditors are used, even time constants of days cannot be maintained, let alone time constants of hours, minutes, seconds or even less that are contemplated in accordance with the presently disclosed subject matter.

It is to be further noted that in some cases only parts of the data used for calculating the welfare score or components thereof are measured on a periodical basis (e.g., every minute, every 10 minutes, etc.), or continuously or near continuously, however due to the fact that each such newly acquired measurement can have an effect on the welfare score and/or components thereof, the welfare score and/or components thereof can be recalculated whenever new measurements are obtained, thus keeping the welfare score and/or components thereof updated in a similar periodical basis, or continuously or near continuously.

In some cases, at least some of the data relating to the member is extracted by analyzing optical information, e.g., images and/or videos, that include at least part of the member. Optical analysis can be used to determine the member's external impression, to assess the relationship between the member and its caregiver and even to determine the location of the member in relation to known landmarks in the image/video.

Utilization of the monitoring data allows for an objective, universal welfare assessment that can at least complement, if not replace, subjective human audits. In addition, utilization of the monitoring data can enable a real-time or near real-time automatic animal welfare assessment based on the collected monitoring data that can be collected in real-time or near real-time. In addition, or as an alternative, as further detailed herein, inter alia with reference to Figs. 10 and 11, utilization of the monitoring data allows estimating an amount of greenhouse gas emission, optionally in real-time or near real-time, based on the collected monitoring data that can be collected in real-time or near real-time.

Monitoring devices gather at least some of the data relating to the animal population by monitoring the members' parameters over time. In some cases, at least part of the monitoring devices can be attached to a given member (e.g., in the form of a monitoring tag, a monitoring collar, etc.) thereby collecting (continuously, or near continuously, or periodically, or in some combination (where some data is collected periodically and some data is collected continuously or near continuously)) the given member's monitoring data.

Additionally, or alternatively, at least part of the monitoring devices may not be attached to a specific member, but be positioned in locations that allow for the monitoring of one or more members either simultaneously or sequentially. For example, at least part of the monitoring devices can be manual monitoring devices (operated by a human operator), stationary monitoring devices (e.g., monitoring members as they pass through a gate), autonomously driven monitoring devices (e.g., carried by autonomous drones), monitoring devices incorporated into a mobile device or into a computer, etc.

It is to be noted that at least part of the data gathered by the monitoring devices is associated with a respective member from which it was gathered. For this purpose, in some cases, at least some of the members of the animal population, and in some cases all members of the animal population, can have an identification (ID) means (such as, but not limited to, an ID tag such as an EID tag or a visual ID tag) attached thereto or otherwise associated therewith.

The ID means can be a tag that uniquely identifies the member of the animal population to which it is attached. In some cases, the ID means is part of a monitoring device attached to the member. However, the ID means may alternatively be another type of device used to identify the member using known and/or proprietary methods and/or techniques (including electronic identification, visual identification, camera-based identification, facial or body part recognition, barcode, identification marks, etc.).

As discussed, each member of the animal population can be identified using various systems/devices/methods/techniques, including an ID tag, identification marks (e.g., digits, letters, symbols, or any combination thereof, etc.), readable barcodes, facial (or other body parts) recognition, etc. When read by a suitable reading device (e.g., an ID tag reader), the ID means enables determining at least a unique animal identifier, uniquely identifying the member in question.

It is to be noted that in some cases, one or more of the identification methods may be passive identification methods, such as passive tags. For example, the tags can be a printing of a certain identifier on the animal to be identified, or they can be stickers of a certain visual identifier attached to the animal to be identified, or they can be markings painted on the animal to be identified. In such cases, where the tags are visually identifiable (e.g., barcodes), the respective tag reading device can be, for example, a camera capable of acquiring images in the spectrum of the visual tags.

The monitoring data gathered by the monitoring devices may be stored in records within a data repository. Each record may be associated with a respective member of the animal population and can contain different types of data, including descriptive data, monitoring data, a combination of descriptive data and monitored data, and optionally other types of data.

For example, a record may contain (a) descriptive data (e.g., age of the respective member, sex of the respective member, treatment history of the respective member, genetic information associated with the respective member, etc.) that was not necessarily gathered by the monitoring devices, and (b) monitoring data (e.g. a body temperature of the respective member, movement time of the respective member, feeding time of the respective member, socialism time of the respective member, respiration levels of the respective member, rumination time of the respective member, etc.).

As indicated above, such monitoring data can be monitored continuously, or near continuously, or periodically, or in some combination (where some data is collected periodically and some data is collected continuously or near continuously), using the monitoring devices. Accordingly, when the monitoring data is monitored in a continuous, near continuous, or periodical manner, or in some combination (where some data is collected periodically and some data is collected continuously or near continuously), the respective records can be updated accordingly (e.g., by updating an existing record associated with the respective member, or by adding an additional record indicating the newly acquired values). In some cases, the updating of at least some of the parameters forming a record associated with a respective member can be by averaging the values of the respective parameter/s over time or by performing any mathematical and/or statistical calculation thereon.

Before continuing the description of Fig. 1, it is to be noted that the monitoring data can be alternatively, or additionally, utilized in order to estimate an amount of greenhouse gas emission, optionally in real-time or near real-time, as further detailed herein, inter alia with reference to Figs. 10 and 11.

Attention is now drawn to Fig. 1 and to the calculation of health score 120 for a given member of the animal population, which can form part of the welfare score calculation, as indicated herein. The health score 120 for a given member of the animal population is determined based on criteria of basic health and functioning of the given member, including, specifically, freedom from disease and injury. In some embodiments, health score 120 can be calculated based, at least in part, on identifying symptoms that can be associated with health-related issues (e.g., illness, injury, etc.). In some cases, the health score 120 is determined based on finding statistically significant correlations between the identified symptoms and illnesses or injuries. In some cases, the strengths of such correlations between the symptoms that are indicative of illness and/or injuries is also used in order to determine the health score 120.

Those symptoms can be identified by monitoring rumination and/or energy levels of an individual member, of a group of members of the animal population or, of the entire animal population. Additionally, or alternatively, the symptoms can be identified by based, at least in part, on monitoring visual indicators associated with health problems and/or monitoring breathing patterns of an individual member, of a group of members of the animal population or, of the entire animal population.

Energy level can be measured, for example, by detecting movements of a given member over a given time period (e.g., a day) and deducing the amount of energy required from the given member to perform these movements (optionally based on specific characteristics of the given animal). The movements can be detected by monitoring an acceleration signal obtained for example from an accelerometer comprised within a monitoring device attached to the given member.

Rumination can be measured, for example, by detecting ruminating activity of a given member over a given time period (e.g., a day). This can be achieved by analyzing acceleration signal obtained for example from an accelerometer comprised within a monitoring device attached to the given member and detecting signals that correlate to reference signals that are known to be associated with rumination activity.

Breathing patterns can be determined, for example, by detecting various breathing patterns of a given member over a given time period (e.g., a day). This can be achieved by analyzing acceleration signal obtained for example from an accelerometer comprised within a monitoring device attached to the given member and detecting signals that correlate to reference signals that are known to be associated with specific breathing patterns. This can enable determining whether the given animal demonstrates suspicious behavior (e.g., if it is breathing heavily more than usual and/or over a predetermined threshold, etc.).

Visual indicators associated with health problems can be identified using image analysis of images and/or videos of the members of the animal population. For example, scratching or irritation that are correlated with morbidity symptoms can be identified using image and/or video analysis.

It is to be noted that these are mere examples for determination of symptoms that can be associated with health-related issues, and additional and/or alternative data can be used to determine the above indicated symptoms, or other symptoms that can be associated with health-related issues.

The health score 120 of the members of the animal population can be used for calculating a health index indicative of the overall health level of the members of the animal population. In some cases, the health index can be used for identifying morbidity levels in the animal population. For example, a health index showing that more than a threshold (e.g., around 5%) of the animals in the animal population are having health scores 120 below a threshold can be an indication of a developing health problem within the animal population. A health index showing that more than a second threshold (e.g., around 10%) of the animal population are having health scores 120 below a threshold can be an indication of a prevailing health problem. A health index showing that more than a third threshold (e.g., around 20%) of the animal population are having health scores 120 below a threshold can be indicative of a pandemic.

It is to be noted that analysis of the health scores 120 can enable identification of given animals of the animal population that have a certain health issue (e.g., illness, lameness, estrus, etc.). In some cases, analysis of the health index (generated based on the health scores 120 of the members of the animal population) can enable, at the population level, identifying whether the overall health level of the animal population is above an acceptable predetermined health threshold.

It is to be further noted, as further detailed herein, inter alia with reference to Figs. 10 and 11, that in some cases, the health scores 120 and/or components thereof (e.g. rumination time) can be used in the estimation of greenhouse gas emission.

Attention is now drawn to the calculation of affectivity/happiness score 130 for a given member of the animal population. The calculation of affectivity/happiness score 130 for a given member of the animal population can be based on a state the given member is in, for example: pain, distress, frustration, pleasure, etc. Affectivity/happiness score 130 can reflect how the given animal is affected by its environment and its experiences (is it positively affected by its environment or negatively affected by its environment). In some cases, the calculation of affectivity/happiness score 130 for a given member of the animal population is based on level of suffering of the given animal, so that the more suffering is identified - the lower its affectivity/happiness score 130 is.

The state of the given member, or its level of suffering, can be determined, for example, based on changes in its breathing patterns, its rumination activity, its eating activity, or some combination thereof. For example: measuring the variability of rumination and eating times, and levels of heavy breathing between members of an animal population, optionally on a daily basis, can provide a basis for the affectivity/happiness score 130.

One specific exemplary measure based on which the affectivity/happiness score 130 can be determined is the consistency of Rumination Variability (RV) for a period of ten days within the animal population. This analysis indicates that reduced ration digestibility can result in increased risk for metabolic issues and decreasing consistency and that optimal intake will be reflected by consistent, low levels of RV.

Another measure based on which the affectivity/happiness score 130 can be determined is heavy breathing. Analysis of the breathing levels of members of the animal population indicates that heavy breathing is indicative of heat stress (discomfort). It is to be noted that when analyzing heavy breathing behavior on a group level, increased heavy breathing implies overall heat stress amongst the animals in the animal population.

One non-limiting exemplary situation that impacts the affectivity/happiness score 130 for a population of ruminating animals includes increased competition between a given group of members of the ruminating animals population (for example: as a result of lack of bunk space resulting in increased competition for food) that will result in higher variability of rumination and eating times between the members of the given group and will result in a lower affectivity/happiness score 130 for that group of members of the ruminating animals.

Additional examples of circumstances that impact affectivity/happiness score 130 are: bedding conditions of members of the animals population, lighting conditions, square feet per meter per member of the animal population (so as not to overstock the animals), water and feed availability (for example: queuing in front of the water trough is a sign that not all the members of the animal population receive their needs), results of spectrographs of milk of the members of the animal population (for milk giving animals), image analysis of feed and feeding area, feed that is taken only by the more dominant members of the animal population can suggest an issue with levels of feed provided and/or a social problem between the members of the animal population.

In some cases, problems may be inferred from the affectivity/happiness score 130. For example, finding inconsistencies or increasing differences between the members of the animal population in an affectivity/happiness score 130 that is based on the housing conditions for the animals may lead to a conclusion that there is a problem in the bedding conditions (which is part of the housing conditions) for these members.

It is to be further noted, as further detailed herein, inter alia with reference to Figs. 10 and 11, that in some cases, the affectivity/happiness score 130 and/or components thereof (e.g., the consistency of Rumination Variability (RV)) can be used in the estimation of greenhouse gas emission.

Turning to the natural living score 140, the calculation thereof is based on analysis of behaviors indicative of the ability of the given animal to live a reasonably natural life by carrying out natural behavior and having access to natural elements in its environment. These specific behaviors can include: eating activity, grazing activity, activity level (e.g., the quantity and intensity of movement of the given animal), walking activity, etc. These behaviors can be used to verify that animals have, for example, enough time and opportunity to eat, express normal high-levels of activity, are not forced to walk to much, or are restricted from appropriate amounts of movement/walking. A non-limiting example related to the natural living behavior for dairy cows is in a dairy farm wherein calves may be regularly separated from their mothers within the first day after birth, and are fed milk from a bucket, usually twice per day. With such infrequent meals the total milk intake is limited so that the calf does not receive too much milk at one time. By contrast, under natural conditions, cows stay fairly close to the calves for the first two weeks, and the calf will feed many times per day in smaller meals. Adjusting the feeding systems to correspond more closely to the animals' natural behavior (staying close to the mother and feeding often but in relatively smaller amounts) will result in a higher natural living score 140 for that calf. An additional example related to the natural living behavior is the time spent by a grazing animal in the pasture looking for food that can suggest a need for fencing reallocation.

It is to be further noted, as further detailed herein, inter alia with reference to Figs. 10 and 11, that in some cases, the natural living score 140 and/or components thereof (e.g., measurements of the grazing activity) can be used in the estimation of greenhouse gas emission.

As further detailed herein, A single welfare KPI (e.g., one of the: health score 120, affectivity/happiness score 130 or natural living score 140), or any combination of welfare KPIs, can be utilized to determine the welfare of the animal population, or of a sub-group within the population, or of a single member of the animal population and optionally to suggest an action to be taken upon the member/s of the animal population. Such actions can include treating the member(s), moving the member(s), examining the member(s), separating the member(s) from the remainder of the animal population, changing the feeding conditions of at least some of the members, changing the water conditions of at least some of the members, changing the bedding conditions of at least some of the members, etc.

Automatic determination of the welfare of members of an animal population, using the teachings herein, can enable calculating a welfare score without the need for presence of a human auditor, and in time intervals that are impossible for a human operator (and optionally even in real time or near real-time). The welfare KPI scores can also take into account large amounts of data, including historical trends, whereas human auditing is limited in the human's ability to gather and obtain insights from large amounts of data. Still further, due to the fact that the welfare KPIs score determination is not made by humans it does not suffer from human bias. Personal impression and auditing proficiency have no effect on the automatic welfare KPIs score determination in accordance with the teachings herein. It is to be noted that in some cases, audits are made on a six-monthly basis, or even in longer time intervals, and clearly in such situations issues may arise that will simply be unnoticed and unassessed for six months.

The automatic determination of animal welfare disclosed herein can also be used to improve the wellbeing of a given animal or groups of animals of the animal population in real time, or near real time. For example, the system and method disclosed herein can enable identifying declining animal conditions of a single animal or a group of animals of the animal population. Further, the system and method disclosed herein can provide the animal caregiver and/or a regulator (such as a governmental organization) with information that can be used to take action in real or near real time to improve animal welfare, such as by obtaining treatments for veterinary or health issues such as: infectious diseases, ectoparasites, and/or reproductive issues.

Moreover, based on the system and method disclosed herein, benchmarks can be determined for one or more locations or facilities of animal populations. These benchmarks can be used as references over time to automatically determine the adherence of the facilities or locations with an expected level of animal welfare. These benchmarks can be measured and adjusted, optionally continuously.

Still further, the system and method disclosed herein can utilize objective, automatic and optionally continuously, collected information to empirically determine welfare scores that can enhance or replace the traditional human auditing method of animal welfare. By that, the system and method disclosed herein can provide a non-biased, fraud-free, universal model for evaluating animal welfare.

The animal welfare disclosed herein can be used to set standards (optionally industry wide standards), benchmark between different animal populations and/or between groups of members of one or more animal populations, and provide management or veterinarian support, optionally in real time, to mitigate and reduce emerging risks. The animal welfare disclosed herein can also be used by insurance and/or finance providers to evaluate risks in insuring and/or financing related to the animal population.

For example, the animal welfare (including the animal welfare KPI scores) disclosed herein can be used to set standards for minimal animal welfare for various types of animal caregivers, whether institutional (such as farms in which cows, swine, equine or any other type of animal is maintained, for slaughter, milk production or for any other purpose) or private animal owners (such as pet owners). Having such standards can enable regulation (enabling governments or other regulators make sure that animal welfare meets the required standards, and quickly identify any irregularity), value and/or risk estimation (enabling insurers, lenders or other type of financial institutions, trade associations, customers, or any other entity that is interested in valuation and/or risk estimation of the animals or their products, to more accurately evaluate the animals or their products such as milk, meat, etc.), and more. For example, the improved objectivity, consistency, frequency, and reliability of the welfare systems and methods descried herein allows for more accurate and precise comparison between disparate farms/animal facilities, which is one of the advantages that enables entities to generate effective standard setting, risk estimation regulation, etc.

In some cases, the animal welfare score can also be used as a measure based on which a certification of animal welfare can be provided, similarly to the Marine Stewardship Council (MSC) Certification used in the fishing industry.

It is to be noted that the ability of the system disclosed herein to monitor the animal welfare (utilizing the animal welfare KPI scores) of animal populations in a continuous (or near continuous) reliable, consistent, and objective manner, is extremely powerful and important. The monitoring described herein enables all stakeholders (animal caregivers, regulators, financial institutions, trade associations, milk processors, meat processors, retail chains, end-customers, or any other entity that may have an interest in the animals' welfare) to receive relevant information for making decisions in real-time, or almost in real-time (e.g., within milliseconds, seconds, minutes, hours or days) based on continuously, or near continuously, updated reliable, consistent, and objective information (as opposed to existing solutions in which such information is unavailable). A few non-limiting examples for uses of the animal welfare according with the presently disclosed subject matter: financial institutions can evaluate risks based on relevant information indicative of the animal welfare at the time they make decisions; Regulators can act upon detecting an irregularity when the irregular situation is detected and not in retrospect; retail chains, milk processors, meat processors, end-customers, or any other consumers of products of the animal population can get information of the animal welfare of the animal population from which the products originate; veterinarians can proactively act upon an identified need based on the animal welfare; diary and/or meat producers can compare farms based on the animal welfare, so they can make informed purchasing decisions.

In some cases, the welfare score can be determined also based on a sustainability index and/or based on a greenhouse gases index. In such cases, the information based on which such index/es are determined can be obtained using sensors that gather relevant information (e.g., water and/or energy resources use, waste generation, freshwater use, air gases, etc.), or using the greenhouse gas emission estimation and reduction system and method detailed herein, inter alia with reference to Figs. 10-12.

It is important to note that the welfare score according to the teachings herein is determined based, at least in part, on direct analysis of the animals behavioural and physiological signals and not based on human interpretation.

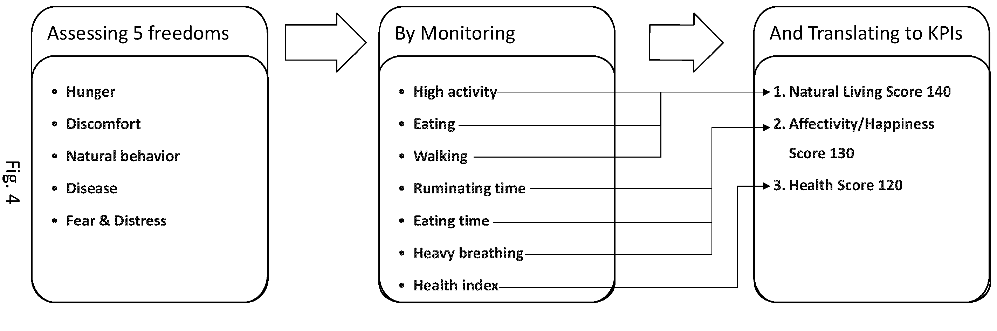

A schematic illustration of a non-limiting example of determining welfare KPI scores for an animal population can be viewed in Fig. 4, wherein the assessment of freedoms of the animal population is achieved by continuously gathering the monitoring data and the descriptive data (as detailed herein) associated with the animal population. The welfare KPI scores are determined based on the gathered data.

It is to be noted that the monitoring data types examples in Fig. 4 are non-limiting examples and that the types of monitoring data may vary for different types of animal population. For example: for companion animals the data that is monitored can include play behavior, socializing with other animals, etc. For livestock the data monitored can include activity level, eating time, walking time, rumination time, heavy breathing, health-related parameters (e.g., body temperature, etc.) living conditions, stock levels, feed time behavior, play behavior, socializing with other animals, etc.

It is to be further noted that companion animals' welfare is viewed more on the individual animal level, while livestock welfare is viewed more on the population level.

In some cases, the animal population can be located at one or more facilities (e.g., farms, enclosures, etc.). In such cases, a welfare score can be calculated on a facility level, by analyzing the welfare KPI scores associated with the members of the animal population located in that facility. For example, different farms can have their own welfare scores allowing comparison between farms.

In addition, the welfare KPI scores can be used to monitor the wellbeing of groups of members of the animal population, thus giving tools to the human care giver of the animals to monitor as well as more economically manage the animal population over time. The welfare KPI scores described herein have many benefits for the animals and the animal care givers. For example, animal management can use the welfare KPI scores internally, for example within a farm, by moving specific members or groups of members between enclosures within the farm, thus achieving hierarchy balancing by managing and removing feeding and social pressure from individual animals or from groups of animals). As indicated herein, the welfare KPI scores can also provide reliable, objective, consistent, and continuous information to all stakeholders (animal caregivers, regulators, financial institutions, trade associations, milk processors, meat processors, retail chains, end-customers, or any other entity that may have an interest in the animals' welfare) for decision making.

As a non-limiting example of utilizing the welfare KPI scores, the scores can help an animal care giver maintains his farm's sustainability by identifying the specific operation segment that requires attention, for example: bedding, feeding, milking, treatment, or any other segment of a farm. The animal care giver in this non-limiting example can monitor overall morbidity of the animal population, observe members that are breaking down due to being pushed beyond their metabolic capacity or being poorly-managed, which may affect their production efficiency and prevent them from reaching their optimal production capacity. The animal care giver can also examine housing conditions, identify historical trends within the animal population and create benchmarks that are continuously measured and adjusted for his farm. The benchmarks can be on the animal welfare score and/or on any one, or any combination of the health score 120, the affectivity/happiness score 130, or the natural living score 140, and, as indicated herein, any one of the benchmarks, or any combination of two or more of the benchmarks can be updated manually, or automatically.

The welfare KPIs can be also used by regulation entities or sourcing companies for monitoring regulated or supplier farms, develop procedures with the animal care giver they regulate or source from, manage public and customer opinion and ensure they maintain transparency around their brand name and brand value.

In addition, the welfare KPI scores can be used to improve the overall health of a given member of the animal population, or groups of animals of the animal population, by identifying a condition and suggesting treatments for veterinary issues even before external indications of illness are detected. As some animals do not externalize sickness symptoms, the welfare KPI can express a change in the animal population that is suggestive of a health problem of some of the members of the animal population. This can enable earlier and more effective treatment of health problems - for example by saving on more invasive drugs and treatments; by starting treatment earlier due to an earlier alert compared to other methods, and this in turn will reduce the number of sick animals, reducing the length of time the animal population is impacted by any health problems, etc. The early recognition of problems can also prevent changes to the member's routine that can lead to discomfort to the member of the animal population. A routine (e.g., feeding times, activity periods, rumination and social behavior schedules, etc.) of a single member and/or a group of members and/or the entire animal population can be identified by analyzing the welfare KPI scores during a specific time period. Once a consistent routine is identified, changes in the welfare KPI scores can be used to identify early warning signs of alterations in the consistent routine before a collapse of that routine.

For example, if a given group of members of an animal population has a health score 120 below a specific threshold (for example: a health score 120 that indicates a percentage of sick members of the animal population is greater or equal to 10% of the population) and a natural living score 140 below a second threshold (for example: the recorded feed availability and/or walking distance covered during the day and/or high activity levels are a threshold percentage over or under historical levels), this measurement can be indicative of an infectious disease problem within the population (e.g., respiratory diseases, gastro-intestinal diseases or reproductive diseases).

These infectious diseases for cows can include, for example, one or more of: Respiratory diseases where the pathogens involved are bacteria, among others: Mannheimia haemolytica, Pasteurella multocida, Mycoplasma bovis, and Histophilus somni; and viruses, amongst others: bovine coronavirus (BCV), parainfluenza-3 (PI3) virus, bovine respiratory syncytial virus (BRSV), bovine viral diarrhoea virus (BVDV) and bovine herpes virus 1 (BHV1), which causes infectious bovine rhinotracheitis (IBR). Genera diseases, where additional immunoactive components are based on, or derived from, micro-organisms that are pathogenic to ruminants. Examples of such micro-organisms are: for bovines (the bovine animal is taurine cattle (Bos taurus), zebu cattle (Bos indicus), buffalo, bison, yak, or wisent): Neospora spec., Dictyocaulus spec., Cryptosporidium spec., Ostertagia spec., bovine rotavirus, bovine viral diarrhoea virus, bovine coronavirus, infectious bovine rhinotracheitis virus (bovine herpes virus 1), bovine paramyxo virus, bovine parainfluenza virus, bovine respiratory syncytial virus, rabies virus, bluetongue virus, E. coli, Salmonella spec., Staphylococcus spec., Mycobacterium spec., Trueperella spec., Brucella spec., Clostridia spec., Pasteurella spec., Mannheimia spec., Haemophilus spec., Leptospira spec., and Fusobacterium spec., Mycobacterium bovis, and Trueperella pyogenes. For sheep and goats (caprine, ovine): Toxoplasma gondii, peste des petit ruminant virus, bluetongue virus, Schmallenberg virus, Mycobacterium spec., Brucella spec., Clostridia spec., Coxiella spec., E. coli, Chlamydia spec., Clostridia spec., Pasteurella spec., and Mannheimia spec. For cervines: Epizootic haemorrhagic disease virus, bluetongue virus, papilloma virus, Borrelia burgdorferi, Mycobacterium bovis, and Trueperella pyogenes. Reproductive diseases such as: Mastitis: Staph and Strep, fertility metritis: Trueperella pyogenes, E. coli, and Fusobact. necrophorum.

The infectious diseases of swine can include, for example, one or more of enteric pathogens which include Salmonella spp., in particular S. typhimurium, S. choleraesuis; Lawsonia intracellularis; Astroviruses; Rotavirus; Transmissible gastroenteritis virus; Brachyspira spp, in particular B. hyodysenteriae, B. pilosicoli; Clostridium spp., in particular C. difficile, C. perfringens types A, B and C, C. novyi, C. septicum, C. tetani; Porcine enteric picornaviruses; Porcine enteric caliciviruses ;respiratory pathogens, which include: Actinobacillus pleuropneumonia; Bordetella bronchiseptica; Erysipelothrix rhsiopathiae; Haemophilus parasuis, in particular subtypes 1, 7 and 14; Pasteurella spp., in particular P. multocida; Mycoplasma spp., in particular M. hyopneumoniae, M. hyorhinis; Swine influenza virus; PRRS virus; PED virus; Porcine circovirus, in particular PCV2 and PCV3; Porcine parvovirus; Pseudorabies virus; Eperythrozoonosis suis, Mycobacterium spp., in particular M. avium, M. intracellulare, M. bovis; Porcine respiratory corona virus; Arcanobacterium pyogenes; Porcine adenovirus; Clasical swine fever; Porcine cytomegalovirus; African swine fever virus; or other pathogens, which include Escherichia coli, Streptococcus spp., in particular S. suis, S. porcinus, S. dysgalactiae, preferably subsp. equisimilis; Brucella suis, in particular biovars 1, 2 and 3; Leptospira spp., in particular L. australis, L. canicola, L. grippotyphosa, L. pomona, L. icterohaemorrhagicae, L. interrogans, L. tarassovi, L. hardjo, L. sejroe; Encephalomyocarditis viruss; Hemagglutinating encephalomyelitis virus; Japanese encephalitis virus; West Nile virus; Reovirus; Rubulavirus; Menangle virus; Nipah virus; Vesicular stomatitis virus; Virus of vesicular exanthema of swine; Swine pox virus; Swine herpes virus; and Staphylococcus hyicus.

An action can then be taken, for example, to treat the situation, as further detailed herein, inter alia with reference to Fig. 3.An action can be suggested based on the overall welfare score and/or the health score 120, and/or the affectivity/happiness score 130 and/or the natural score 140 of the animals within the population.

Having described the components of the welfare score, attention is now drawn to Fig. 2. Fig. 2 is a block diagram schematically illustrating one example of a welfare determination system for determining the welfare of an animal population, in accordance with the presently disclosed subject matter.

Welfare determination system 200 comprises a network interface 220 (e.g., a network card, a WiFi client, a LiFi client, 3G/4G client, or any other component), enabling welfare determination system 200 to communicate over a network with external systems from which it obtains monitored parameters of members of an animal population and/or descriptive data associated with the members. The external systems can be monitoring devices configured to monitor parameters of the members, or any other intermediate system(s) that obtain the information about the members from the monitoring devices (e.g., computerized systems that manage at least part of the members of the animal population).

Welfare determination system 200 further comprises, or is otherwise associated with, a data repository 210 (e.g., a database, a storage system, a memory including Read Only Memory - ROM, Random Access Memory - RAM, or any other type of memory, etc.) configured to store data, optionally including, inter alia, animal information records. Each information record is associated with a distinct member of the animal population and can include descriptive data of the member (e.g., age of the distinct member, sex of the distinct member, treatment history of the distinct member, genetic information associated with the distinct member, etc.) and monitoring data and/or monitored parameters of the distinct member as monitored by the monitoring devices over time (e.g., health parameters, behavioral parameters, affectivity/happiness parameters, etc.). Data repository 210 can be further configured to enable retrieval and/or update and/or deletion of the stored data. It is to be noted that in some cases, data repository 210 can be distributed, while the Welfare determination system 200 has access to the information stored thereon, e.g., via a wired or wireless network to which Welfare determination system 200 is able to connect (utilizing its network interface 220).

Welfare determination system 200 further comprises a processing circuitry 230. Processing circuitry 230 can be one or more processing units (e.g., central processing units), microprocessors, microcontrollers (e.g., microcontroller units (MCUs)) or any other computing devices or modules, including multiple and/or parallel and/or distributed processing units, which are adapted to independently or cooperatively process data for controlling relevant welfare determination system 200 resources and for enabling operations related to welfare determination system's 200 resources.

Processing circuitry 230 can comprise a welfare KPIs determination module 240. Welfare KPIs determination module 240, can be configured to determine the welfare KPIs for an animal population (or a group within that population), as further detailed herein, inter alia with reference to Fig. 3.

Turning to Fig. 3, there is shown a flowchart illustrating one example of a sequence of operations carried out for determining the welfare of an animal population, in accordance with the presently disclosed subject matter.

In accordance with the presently disclosed subject matter, welfare determination system 200 can be configured to perform welfare KPIs determination process 300 (optionally in real-time or near-real time), e.g., using welfare KPIs determination module 240.

As indicated herein, with reference to Fig. 1, the welfare KPIs are determined based on data associated with members of the animal population. The data can originate from various sources, from within the system and/or from external systems. For example: some of the data can be descriptive data (e.g., age of the distinct member, sex of the distinct member, treatment history of the distinct member, genetic information associated with the distinct member, etc.) and some of the data can be monitoring data, e.g., parameters of the animal (for example: temperature, movement time, feeding time, social behavior time, respiration levels, rumination time, etc.) that are monitored over time using one or more monitoring devices.

The data gathered from the monitoring devices is stored in records within a data repository. Each record is associated with a respective member of the animal population and holds descriptive data (e.g., age of the respective member, sex of the respective member, treatment history of the respective member, genetic information associated with the respective member, etc.) and monitoring data, e.g., parameters of the animal (for example: temperature, movement time, feeding time, social behavior time, respiration levels, rumination time, etc.) obtained from the monitoring devices. Monitoring data may be collected continuously, or near continuously, or periodically, or in some combination (where some data is collected periodically and some data is collected continuously or near continuously).

Welfare determination system 200 is configured to obtain at least a subset of the records, the subset being associated with a first group of members of the animal population (block 310). In some cases, all of the records of the animal population are obtained by welfare determination system 200. In other cases, the first group of members can consist of a sub-population of the animal population, selected in accordance with one or more criteria (e.g., sex, age, location, treatment history, etc.). For example: the animal population can be dairy cows within a given farm and the sub-population are all the dairy cows that received a given treatment during the last year. It is to be noted that the animal population can be located in one or more geographical sites and/or within specific locations within these sites (e.g., enclosures, pasture, treatment areas, etc.).

The animals of the animal population can be for example: ruminating animals, livestock, swine, companion animals or any other type of non-human animal.

After obtaining the at least subset of the records, welfare determination system 200 is further configured to calculate, based on the subset of records, for at least part of the members of the first group of members, at least one of: (a) a health score 120 indicative of a health state of the respective member, (b) a natural living score 140, indicative of compliance of a behavior pattern of the respective member with a desired natural behavior pattern, or (c) an affectivity/happiness score 130, indicative of compliance of an affectivity/happiness measure of the respective member with a desired affectivity/happiness measure (block 320).

For the health score 120 calculation, the parameters that can be used can include, for example, the energy level of the given member and/or the time the given member ruminates. Rumination, energy levels, heavy breathing, chewing, core temperature, Image analysis to identify indicators of health problems and optionally additional and/or alternative indicators from the member can determine if the member has health disorders (e.g., illness and/or injury, etc.). The health score 120 calculation can be based on the absolute values of these parameters, on changes of these monitored parameters over time, relative values of these parameters (such as percentages, etc.) or on a combination thereof.

The health score 120 for the given member can be determined, for example, based on the consistency of the values of at least some of the parameters over time (e.g., their consistency over a rolling average for a given time period, such as a ten-day rolling average). In some cases, the consistency of the values can be measured against reference parameters, for example: reference parameters that are measured from reference animals. Actual members of the animal population (other than the given member) or from another population can be used as reference animals. Historical data can also be used as reference. In some cases, the reference can be a theoretical reference of how a hypothetical reference animal should behave.

For the affectivity/happiness score 130 calculation, the parameters that can be used for the calculation can include, for example, one or more of: (a) respiration activity, including respiration level of the given member (e.g., level of respiration suggestive of a panting animal, or heavy breathing) (b) rumination activity, including percentage of rumination time for the given member within a given time period, for example: the time a given member has ruminated during a given time period (e.g. a day), (c) feeding activity, including percentage of feeding time for the given member within a certain time period, for example: the time a given member was feeding during a given time period (e.g. a day), or (d) external impression of the given member (e.g., using automatic image analysis of images and/or videos that include at least part of the given member).

The affectivity/happiness score 130 calculation can be based on the values of these parameters and/or on changes in the values of these monitored parameters over time. The affectivity/happiness score 130 for the given member can be determined, for example, based on the consistency of the values of at least some of the parameters over time (e.g., their consistency over a rolling average for a given time period, such as a ten-day rolling average). In some cases, the consistency of the values can be measured against reference parameters, for example: reference parameters that are measured from reference animals. Actual members of the animal population (other than the given member) or from another population can be used as reference animals. Historical data can also be used as reference. In some cases, the reference can be a theoretical reference of how a hypothetical reference animal should behave.

For the natural living score 140 calculation, the parameters that can be used for the calculation can include, for example, one or more of: (a) movement activity, including percentage of movement time for the given member within a certain time period (for example: the given member was on the move for 20% of the time within a given day or the given animal had a certain amount of high activity movement withing a given time period), (b) feeding activity, including percentage of feeding time for the given member within a certain time period (for example: the given member was feeding 25% of the time within a given day), (c) social activity, including percentage of social behavior time (e.g., time when the given member is interacting with another animal) for the given member within a certain time period (for example: the given member was engaged in social interaction for 10% of the time within a given day), or (d) schedule activity, changes in the schedule of the given member with respect to historical schedules (for example: if four feedings a day are the norm and less or more than that is taken on a given day).

The natural living score 140 calculation can be based on the values of these parameters and/or changes in the values of these monitored parameters over time. The natural living score 140 for the given member can be determined, for example, based on the consistency of the values of at least some of the parameters over time (e.g., the consistency of percentage of movement time during daytime over a rolling average for a given time period, such as a ten-day rolling average). In some cases, the consistency of the values can be measured against reference parameters, for example: reference parameters that are measured from reference animals. Actual members of the animal population (other than the given member) or from another population can be used as reference animals. Historical data can also be used as reference. In some cases, the reference can be a theoretical reference of how a hypothetical reference animal should behave.

It is to be noted that in some cases, the determination of the natural living score 140 and/or of the affectivity/happiness score 130 of the respective member is also based on one or more environmental parameters, indicative of the state of the environment.

Non-limiting examples of such environmental parameters include weather conditions in the area of the animal, geographical location of the animal, ambient temperature around the animal, etc. that can affect the calculation of the natural living score 140 and/or of the affectivity/happiness score 130. For example: on cold days an animal is expected to stay inside and thus have less movement and less social interaction. Thus, system 200 will take the ambient temperature into consideration when determining the natural living score 140 and/or of the affectivity/happiness score 130. A low level of movement on a cold day could be given low weight for example with respect to the weight given to low level of movement on a warmer day.

It is to be noted that the environmental parameters can include one or more general environmental parameters that are not associated with a specific member of the animal population, and/or one or more member-specific environmental parameters that are associated with a specific member of the animal population. In the latter case, the member-specific environmental parameter can be included in the record associated with the specific member of the animal population.

Upon calculation of at least one of the health score 120, the natural living score 140 or the affectivity/happiness score 130 for the members of the first group, welfare determination system 200 calculates a welfare score of the animal population based on any one, or any combination of: (a) the health scores 120 calculated for the first group of members, (b) the natural living scores 140 calculated for a second group of members, or (c) the affectivity/happiness scores 130 calculated for a third group of members (block 330).

In some cases, the welfare score can be a summation of averages of the health scores 120, the natural living scores 140, and the affectivity/happiness scores 130. In some cases, different weights can be assigned to each of the average scores, based on various considerations (e.g., based a level of credibility of the scores used to calculate the respective average, based on a variability of the scores used to calculate the respective average, etc.). In some cases, the welfare score can be normalized to a value between 0-100.

In some cases, the first group, the second group and the third group are identical and the calculation are made using the scores of all the members of the groups. In other cases, the calculation of the welfare score takes into account natural living scores 140 and/or affectivity/happiness scores 130 that have been calculated only for healthy members of the first group, e.g., members having health scores 120 above a threshold.

It is to be noted that in some cases the welfare score for the animal population, or any sub-group thereof, is calculated based on a variation between, or within, the individual scores (e.g., health scores 120, affectivity/happiness scores 130 and natural living scores 140) calculated for the members of the animal population, or the sub-group thereof.

A non-limiting example for the effect of variance within the individual affectivity/happiness scores 130 of the first group of the members, or variables used in its calculation, is that where a variation below one or more threshold ranges (e.g., for cows, one or more of: rumination variability below a range of 16-20% and/or for rumination times - variability below a threshold of 20-30 minutes and/or for heavy breathing - a variability in the levels of breathing below a range of 15-20%, etc.) is considered good (and thus this will not adversely affect the overall welfare score of the animal population), and a variation of more than one or more threshold ranges (e.g., for cows, one or more of: rumination variability above a range of 16-20% and/or for rumination times - variability above a threshold of 20-30 minutes and/or for heavy breathing - a variability in the levels of breathing above a range of 15-20%, etc.) will result in the welfare score being reduced. It is to be noted that in this example reference is made to one or more threshold ranges, however this is by no means limiting, and the variations can be compared with one or more sliding scale calculated based on historical values of the affectivity/happiness scores 130 of the first group of the members, or variables used in its calculation.

It is to be noted that all the calculations mentioned can be optionally completed in real-time or near-real time, e.g., in very short intervals of times, close to instantaneous, or within up to a few seconds. Alternatively, the calculations may take minutes or more to complete.

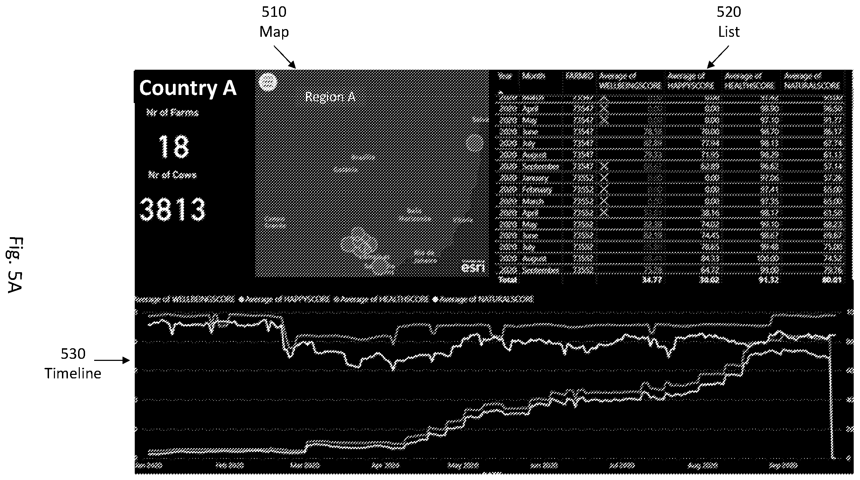

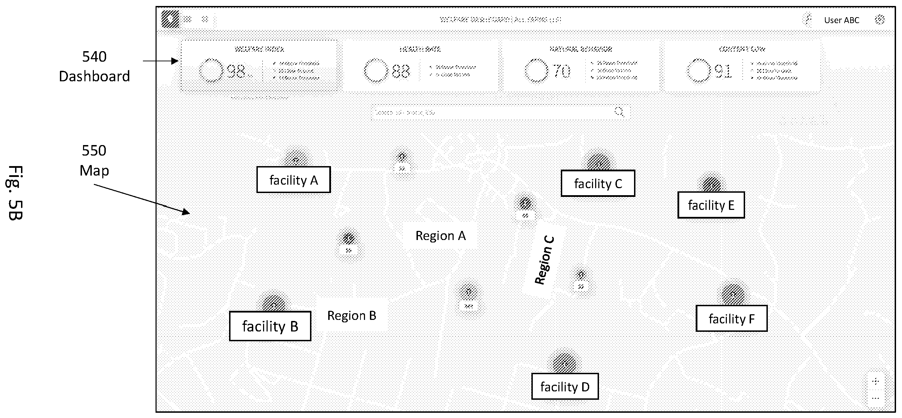

In some cases, the calculated welfare scores for the animal population, or any sub-group thereof, can be presented to a user of system 200 utilizing a Graphical User Interface (GUI). A non-limiting illustration of examples of possible GUI screen used by system 200 are depicted in Figs. 5A and 5B. The GUI can present current (and optionally real-time, or near real-time) and/or historical welfare KPI scores, including graphs and reports based on these current and/or historical welfare KPI scores.

Looking at Fig. 5A, there is shown a non-limiting GUI example, including: a map 510 GUI component, a list 520 GUI component and a timeline 530 GUI component. The map 510 displays one or more facilities (for example: farms) having animal populations that are monitored by welfare determination system 200. The list 520 displays information about the facilities welfare KPI scores within a given timeframe (e.g., a calendric month, year, etc.) in a tabular format. The columns of list 520 include data about each facility within the given timeframe (e.g., average welfare KPI score, average health score 120, average affectivity/happiness score 130, average natural living score 140, etc.) These components interrelate - when a user of system 200 choses one or more facilities using map 510 or list 520, the welfare KPI scores, the health scores 120, the affectivity/happiness scores 130 and the natural living scores 140 for the chosen facilities over the given timeframe are presented within timeline 530. In addition, the GUI can present the user with historical trends of the calculated welfare KPI scores within the timeline 530 GUI component. The user of welfare determination system 200 can use the GUI to generate a presentation of the calculated welfare scores in various timelines and/or regions. The GUI can present the data of a single group or of a number of groups in a comparative way. The one or more groups presented can be associated with one or more facilities so that the GUI can present welfare KPI scores for specific facilities or for groups of facilities. For example: the GUI can present a number of graphs (within timeline 530 GUI component) of the welfare KPI scores for the last year for all facilities in a number of geographical locations selected from map 510, thus giving the user of welfare determination system 200 a tool to easily compare between the welfare KPI scores in different geographical locations.

Looking at Fig. 5B, there is shown another non-limiting GUI example, this example is a dashboard style GUI, including: map 550 GUI component and one or more dashboard 540 GUI components. The dashboard 540 GUI components can display different colors to indicate of the relative welfare KPI scores of animals in one or more locations, as chosen interactively by the user of system 200 from map 550 GUI component. For example: the number of animals with a welfare KPI score above a given threshold is marked green, the number of animals with a welfare KPI score within the given threshold is marked yellow and the number of animals with a welfare KPI score below the given threshold is marked red. Additional dashboard 540 GUI elements can be similarly color-coded to display the information for the health scores 120, the affectivity/happiness scores 130 and the natural living scores 140 for the animals in the chosen locations. In addition, the symbols for the facilities on the map can be similarly color coded. For example, facilities having a welfare KPI score below the given threshold can be marked red, facilities having a welfare KPI score above the given threshold can be marked green and facilities having a welfare KPI score within the given threshold can be marked yellow.

After calculating the welfare score, welfare determination system 200 can be optionally configured to suggest an action to be taken on the animal population based on the welfare score (block 340). The action can be taken on a given member of the animal population, on a given group of members of the animal population (e.g., members of the animal population that are located in a certain location, such as a farm), or on the entire animal population.

The action can be, for example, a recommendation to the animal caregivers to change the human-animal relationship or to change the living environment and/or conditions of the animal/s. The recommendation can additionally, or alternatively, be to a regulator (such as a governmental organization) to perform actions in case the animal welfare is insufficient (noting that in some cases, the regulator can be provided with information on the welfare scores of the animal population/s under its supervision on a periodical or continuous manner, thereby enabling enforcing animal welfare requirements in a reliable, objective, repeatable and non-biased manner). The recommendation can additionally, or alternatively, be to a financial institution to make sure obligations of the animal caregiver to the financial institution are met. The recommendation can additionally, or alternatively, be to retail chains, milk processors, or meat processors, to prefer purchasing products originating from one animal population over other animal populations (based on animal welfare scores calculated for a plurality of animal populations, or for a plurality of groups of animals from a single animal population). The recommendation can additionally, or alternatively, be to veterinarians to proactively act upon identifying a need based on the animal welfare.

In some cases, the action can be executed automatically by an automated treatment system that is able to change the treatment given to the animal/s, upon receiving the suggested action from welfare determination system 200.

It is to be noted that the action can be any action that influences one or more members of the animal population. For example: administering a treatment (vaccinating non-infected members of the animal population, providing antibiotics, antiviral drugs, painkillers, anti-inflammatory drugs, general health boosters like vitamins and hormones like steroids, etc.) to one or more members of the animal population changing the environmental temperature that one or more members of the animal population are exposed to, changing the feeding parameters of one or more members of the animal population, changing a schedule or routine of one or more members of the animal population, changing sleeping parameters of one or more members of the animal population, separating one or more members of the animal population from the remaining members of the animal population, improving feed quality and availability to one or more members of the animal population, lowering house, bunk or both stocking rates, improving the housing/shelter conditions for one or more members of the animal population (e.g., by changing the environmental conditions control, bedding surface, walking routines, etc.), updating the Standard Operating Procedures (SOP) on a farm for better meeting the animal population's need, identifying outbreaks on time and acting accordingly, etc.

The action can be suggested also based on the health scores 120 and/or on the affectivity/happiness scores 130 and/or on the natural living scores 140 of the members of the animal population or a sub-group thereof.

The actions can be suggested based on determination of potential causes for a lower than desired welfare score, e.g., using rules that correlate potential causes to the welfare score and/or components thereof (the health scores 120 and/or on the affectivity/happiness scores 130 and/or on the natural living scores 140 of the members of the animal population or a sub-group thereof). As can be appreciated, correlations exist between certain potential causes (feed issues, bedding, social pressure, ambient temperature, production pressure) and lower welfare scores (or lower health scores 120 and/or affectivity/happiness scores 130 and/or natural living scores 140). Accordingly, welfare determination system 200 can advise the animal care giver, based on these scores, to take one or more actions, along with a reasoning for providing such advice.

The potential causes can be determined by correlating behaviors of members of the animal population to circumstances or conditions (e.g., environmental conditions, operational conditions, human-relationship conditions, etc.) that impact one of the scores (the health scores 120 and/or on the affectivity/happiness scores 130 and/or on the natural living scores 140) described herein. For example, potential causes for increased variability of rumination and eating of members of the animal population can be a nutrition or feed issue, a bedding problem or social pressure the members of the animal population are suffering from. These causes can be correlated to the welfare scores Similarly, plausible causes for heavy breathing and enhanced variability of rumination and eating of members of the animal population can be an ambient temperature that is too high or an illness the members of the animal population are suffering from.

Attention is drawn to a non-limiting example of using the welfare KPIs in addition to the welfare score for suggesting an action: when (a) the welfare score of an animal population is lower than a first threshold, (b) a health score 120 for the animal population is lower than a second threshold, and (c) a natural living score 140 for the animal population is lower than a third threshold, the welfare determination system 200 can suggest an action of an infectious disease inspection of the animal population. Alternatively, if only one, or two, of the welfare score, the health score 120 or the natural living score 140 are below a threshold, the welfare determination system 200 can suggest another action - such as effectuating treatment for a potentially infectious disease.

In some cases, the action is suggested upon the welfare KPI scores being below a welfare threshold. The welfare threshold can be determined based on statistical analysis of historical welfare KPI scores (for example: using box plots, regression analysis, etc.). The historical welfare KPI scores can be used to identify trends within and/or between animal populations. The determination of the welfare threshold can be performed for one or more facilities (e.g., farms, etc.), geographical locations (e.g., territories, countries, etc.), etc.

It is to be noted that in some cases, different locations/territories have different regulatory and/or environmental conditions. Thus, an adjustable welfare threshold can take into consideration local regulation, local climate, local animal needs, local historical welfare scores, and other relevant factors, which results in a more realistic and useful welfare threshold(s) that can be adopted by different local stakeholders at different locations/territories.

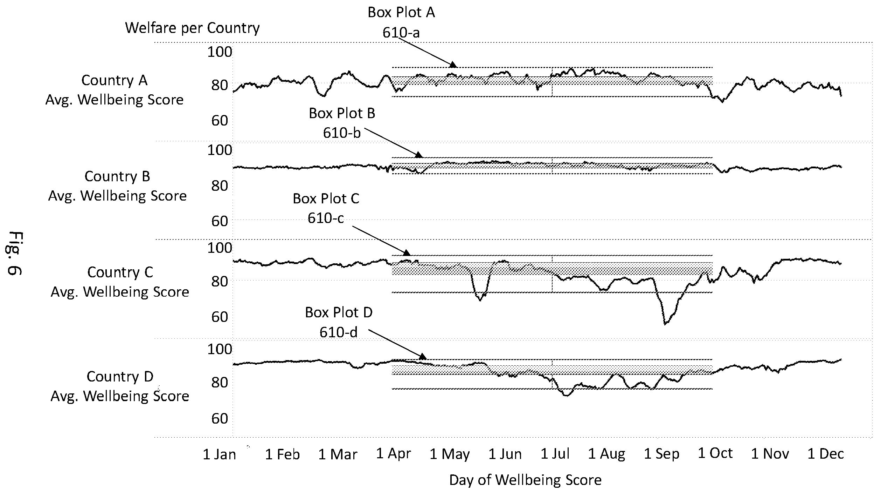

A non-limiting example is shown in Fig. 6, which shows graphs of average welfare scores as determined by welfare determination system 200 over a time-period of a year for animal populations in four different countries. These graphs demonstrate the applicability of the welfare score model used by welfare determination system 200 to various geographical areas, regions and countries having different environmental and seasonal conditions. The behavior of the welfare scores determined by welfare determination system 200 matches known seasonal variations. For example: the dip in the welfare score in country C during May and September coincides with extreme heat conditions measured in that country. The welfare scores in the different countries are used to determine corresponding welfare thresholds. The welfare thresholds for a given geographical area, region or country can be determined statistically based on past welfare scores from that given geographical area, region or country. The statistical determination can be based on average, a mean, a box plot or any other statistical method. A non-limiting example of determining the welfare thresholds for various countries using a box plot (e.g., box plot A 610-a, box plot B 610-b, box plot C 610-c, box plot D 610-d, etc.) method is depicted in the graphs of Fig. 6 - depicting welfare thresholds determined for four different countries during a corresponding given time frame. Box plot is a known statistical method for graphically depicting groups of numerical data through their quartiles. For example: box plot A 610-a has four vertical quartiles (colored white, light gray, dark gray and white). The first quartile, which is the lowest part of box plot A 610-a, holds the median of the lower half of the dataset. The threshold in this example is set to this first quartile - welfare scores that are above the first quartile are above the welfare threshold and welfare scores in the first quartile are below the welfare threshold. This method generated different welfare thresholds for different countries and can be used to determine different welfare thresholds for different time-periods within the same country. It can be appreciated that the welfare thresholds vary between the four countries.

It is to be noted that welfare determination system 200 can set different thresholds for the different locations due to differences between the locations, such as: different animal types, different climate, different customs, different regulation and rules, different laws, different demands on the animals. Welfare determination system's 200 continuous and constant monitoring enables a user of welfare determination system 200 (such as: an animal care giver) to set appropriate thresholds for his location. Each location can be, for example, a farm, a group of farms, a region, a territory, a country, etc.

It is to be noted that the welfare threshold can also change dynamically (e.g., according to weather) and/or periodically (e.g., seasonally). With reference to seasonal related changes, it is to be noted that analysis of historical welfare score enables deamination of periods of the year where the animal population's welfare scores are influenced from weather conditions, the season of year, or other local variable conditions. It can be readily appreciated that the seasonal change is geography dependent and in may vary between countries, or even between geographical areas within a country.

It is to be noted that welfare determination system 200 can set different thresholds for the different locations due to differences between the locations, such as: different animal types, different climate, different customs, different regulation and rules, different laws, different demands on the animals. Welfare determination system's 200 continuous and constant monitoring enables a user of welfare determination system 200 (such as: an animal care giver) to set appropriate thresholds for his location. Each location can be, for example, a farm, a group of farms, a region, a territory, a country, etc.

It is to be noted that the welfare threshold can also change dynamically (e.g. according to weather) and/or periodically (e.g. seasonally). With reference to seasonal related changes, it is to be noted that analysis of historical welfare KPI score enables deamination of periods of the year where the animal population's welfare KPI scores are influenced from weather conditions, the season of year, or other local variable conditions. It can be readily appreciated that the seasonal change is geography dependent and in may vary between countries, or even between geographical areas within a country.

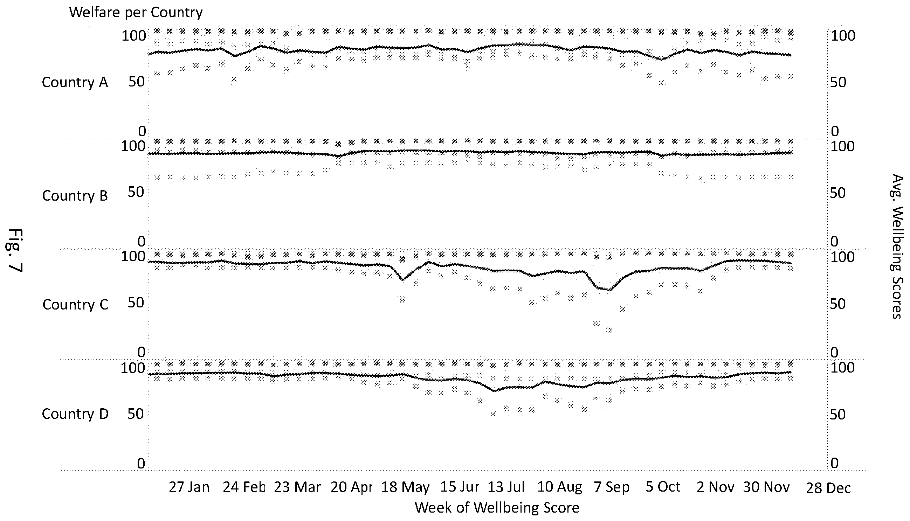

A non-limiting example is shown if Fig. 7, in which data collected from four countries is shown. In "Country A", between January to March and October to December the animals are exposed to conditions that affect the affectivity/happiness score 130 and between April to August there are more issues affecting the natural living score 140. For example, this can be due to tropical climate in "Country A" and the impact of a wet versus dry seasons. In "Country B", between April to October, once animals are allowed into paddocks, the natural living score 140 is improved due to the better conditions compared with the over stocking conditions when housed. The affectivity/happiness score 130 in the same period are osculating more due to feed quality and climate conditions. Further, in "Country C" and "Country D" the graphs depict the impact of extreme conditions: extreme heat stress in May and September pulled the overall welfare KPI score down although the animal environment was not changed. These weather and seasonal effects may be missed by a human auditor, that would clearly not be able to notice smaller and subtler impacts of varying climates, conditions, and environments that can be identified using the welfare determination system 200.

Welfare determination System's 200 welfare KPI scores have been validated against human auditors' welfare KPI scores and the welfare KPI scores generated by system 200 have been found to be valid and useful. For example, the welfare scores determined by the welfare determination system 200 showed good correlation with welfare scores on the same animals as determined by a human audit. In addition, the welfare determination system 200 also provided additional insight, more accurate insight, and more complete insight than the human led audits. This provides examples of the improvement of the current disclosure over the prior art.

In a number of exemplary studies, the welfare KPI scores generated by welfare determination system 200 have been blindly compared with human auditing on representative farm operations. One example involved a population of grazing cows. The human auditor gave a low overall welfare KPI score due to the observation of missing facilities and poor feeding conditions. By contrast, system's 200 welfare KPI scores for the same grazing cows was higher. To prove that the welfare determination system's 200 scores were more accurate, a number of indicators have been examined: lack of illness signs, no diseases for that group of cows, lameness was not observed among these cows, constant feed behavior; additionally the Somatic Cell Count (SCC) of the milk of these cows was tested and found to be at constant and appropriate levels, consistent yield levels, all are indicators that the cow's welfare was not low - as indicated by the human auditor. Indeed, because the cows were low yielding cows naturally, they were able to maintain good welfare despite the external conditions judged to be detrimental to welfare by the human auditor. The human auditor was not able to directly measure the cow's welfare and judge if these external conditions were adversely impacting the welfare. By contrast, the more direct measurement of the animal's welfare, using the welfare determination system 200, was able to achieve this. Thus, this is one example of the welfare determination system 200 described herein providing a more accurate determination of welfare of the cows on the farm.

Another example included a population of high yielding cows that were fed on a Total Mixed Ration (TMR) diet. The human auditor found missing housing facilities, problematic stocking rates and cows that were dirty and thus gave a low welfare KPI score. Welfare determination System's 200 welfare KPI scores for the same high yielding cows was relatively high since welfare determination system 200 took into consideration the consistent TMR, the sufficient bunk space, the efficient cooling solution provided to the cows and the acceptable health score 120 result. As animals' responses to enforced conditions is multifactorial it is hard for a human auditor to grasp without more frequent or continuous monitoring. Human auditors usually notice only the external drop in performance of the animal - when it is usually too late to repair the situation. This is another example of the human auditor relying on external observations without having the benefit of the direct and continuous measurement of the cow's welfare as the currently disclosed welfare determination system 200 employs.

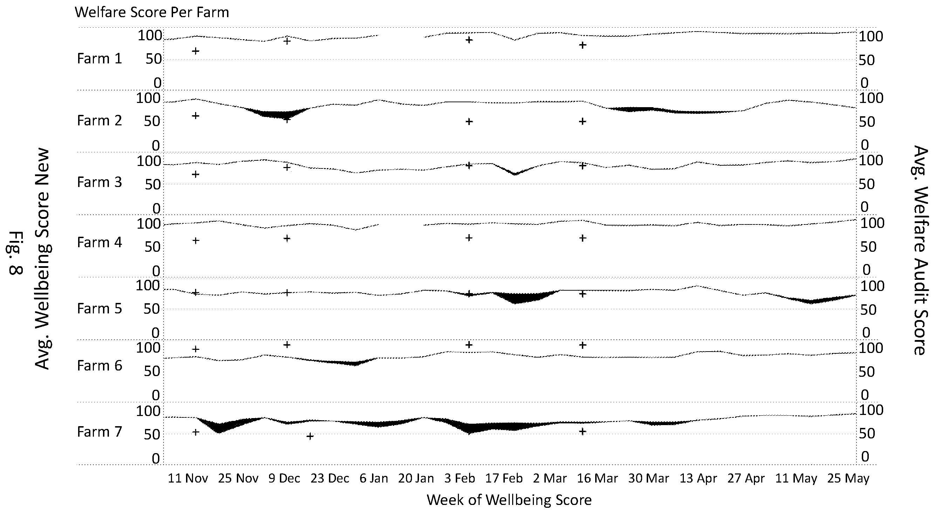

Fig. 8 depicts a number of non-limiting examples of welfare scores as determined by human auditors (marked by +) and welfare determination system's 200 welfare scores (marked by solid lines) at parallel timeframes for a number of given facilities. The areas where the welfare score lines are thicker are associated with the time where the welfare scores were more frequently dropping below the set welfare threshold. It is to be noted that the data in Fig. 8 is actual data from usage of welfare determination system 200 monitoring these facilities, thereby validating the welfare KPI scores generated by system 200.

In some cases, a human auditor may determine a welfare score for a given animal population that is lower than a welfare KPI score determined by welfare determination system 200 for the same given animal population. This can occur because the data and analysis available to the human auditor (e.g., production levels, visual inspections of the animal's environment, etc.) is inferior to welfare determination system's 200 data that can be acquired through continuous monitoring and analysis of the behaviors of members of the animal population (e.g., feeding levels, rumination times, activity and energy levels, etc.) to accurately determine their welfare KPI scores. A higher score given by welfare determination system 200 can also be a result of comparing the measured behavior to a rolling baseline behavior and adjusting the welfare KPI score for the impact of extreme events or slowly deteriorating conditions that are not easily judged by infrequent human audits. Without the ability to compare to a rolling baseline and adjusting for the impact of events such as weather (for example: seasonal weather, extreme weather, etc.), slowly changing conditions (for example: a slow decline is animals' facilities), or subtle changes over time, the human auditor may determine a lower welfare score than the one determined by welfare determination system 200 for the same animal population under the same conditions. In a real-life example, a farm which received a medium-low welfare score from a human auditor due to unclean animal living quarters, low food supplies and low production levels, received a high welfare KPI score from welfare determination system 200 which was in-synch with the animals' actual welfare - the animals had low pressure due to the low productivity expectations and the living quarters actually gave the animals an opportunity to rest and to cool themselves.

A non-limiting example of where the audited score is above the welfare KPI scores calculated by welfare determination system 200 is when environmental conditions change (such as: extreme weather conditions) but the animal living conditions haven't changed. The human auditor score is biased towards the living conditions viewed by the auditor, and thus gives a high auditing score. Welfare determination system's 200 monitoring (that is optionally continuous) senses the effects of the change in the environmental conditions on the animals and thus gives a lower welfare KPI score that is more aligned with the reality of the animals.

Another non limiting example of where the audited score is above the welfare KPI scores calculated by welfare determination system 200 is when the animals are under high physiological pressure (e.g., high production pressure, high workload pressure, etc.). A human auditor is impressed with the yield of the animals and by the farm's physical conditions, but system 200 continuously monitoring of the animals' behaviour detects the high pressure and the animals' reaction to that pressure that results in a low welfare KPI score.

The areas of disagreement between the human auditing welfare scores and system's 200 welfare KPI scores that are based on automatic, and optionally continuous, monitoring, derive from welfare determination system 200 taking into account also the historical trends whereas human auditing is based on an evaluation performed during the time of the audit only. There is no human bias in welfare determination system's 200 welfare KPI score determination - no personal impression, no repeatability problems and no auditing proficiency. Welfare determination system 200 judges the welfare conditions from the animal's physiological signals and not by human interpretation. Welfare determination system 200 can provide higher sensitivity for chronic states such as overstocking, or extreme conditions such as heavy heat stress. Welfare determination system 200 can provide higher specificity in situations where animals can manage the enforced environment due to less depended production or conditions.

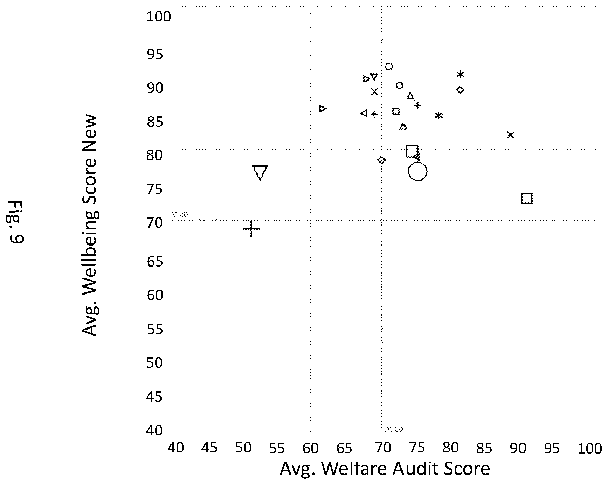

Fig. 9 depicts the consistency between human auditors determined welfare scores and welfare KPI scores determined by welfare determination system 200. The upper right part and the lower left part of the graph in Fig. 9 (where most of the validation measurements are found) is where there is general agreement between the human auditor's welfare scores and the welfare KPI scores determined by welfare determination system 200. The size of the icon in the graph is associated with the welfare KPI scores that are more frequently dropping below the welfare threshold.

Human auditing is limited due to inconsistencies, reputability and precision problems. Welfare determination system 200 mitigates these limitations by providing an accurate and optionally continuous welfare KPI score.

These comparative studies have found that as result of the optionally 24/7 coverage of welfare determination system 200 and the individual monitoring performance of the calculated welfare KPI scores, system 200 delivers a more truthful representation of the reality of the animal population. For example, the welfare determination system 200 is better equipped to deal with scenarios like: uneven operational consistency, extreme weather conditions, oscillating morbidity, and more. The studies have found that production demand has a significant impact on the welfare reflection, i.e., lower production makes harder conditions more tolerated. Based on proven insights, the calculated monitoring welfare score provides an objective (non-human interpreted) view of the animal population condition.

It is to be noted, with reference to Fig. 3, that some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

Having described the welfare determination system for determining the welfare of an animal population, attention is now drawn to **Fig. 10.** Fig. 10 is block diagram schematically illustrating one example of a greenhouse gas emission estimation and reduction system, in accordance with the presently disclosed subject matter. Greenhouse gas emission estimation and reduction system can be configured to estimate greenhouse gas emission in an environment housing an animal population, as further detailed herein.

Greenhouse gas emission estimation and reduction system 700 comprises a network interface 720 (e.g., a network card, a WiFi client, a LiFi client, 3G/4G client, or any other component), enabling greenhouse gas emission estimation and reduction system 700 to communicate over a network with external systems from which it obtains monitored parameters of members of an animal population and/or descriptive data associated with the members. The external systems can be monitoring devices configured to monitor parameters of the members, or any other intermediate system(s) that obtain the information about the members from the monitoring devices (e.g., computerized systems that manage at least part of the members of the animal population).

Greenhouse gas emission estimation and reduction system 700 further comprises, or is otherwise associated with, a data repository 710 (e.g., a database, a storage system, a memory including Read Only Memory - ROM, Random Access Memory - RAM, or any other type of memory, etc.) configured to store data, optionally including, inter alia, animal information records. Each information record is associated with a distinct member of the animal population and can include descriptive data of the member (e.g., age of the distinct member, sex of the distinct member, treatment history of the distinct member, genetic information associated with the distinct member, etc.) and monitoring data and/or monitored parameters of the distinct member as monitored by the monitoring devices over time (e.g., health parameters, behavioral parameters, affectivity/happiness parameters, etc.). Data repository 710 can be further configured to enable retrieval and/or update and/or deletion of the stored data. It is to be noted that in some cases, data repository 710 can be distributed, while the greenhouse gas emission estimation and reduction system 700 has access to the information stored thereon, e.g., via a wired or wireless network to which greenhouse gas emission estimation and reduction system 700 is able to connect (utilizing its network interface 720).

Greenhouse gas emission estimation and reduction system 700 further comprises a processing circuitry 730. Processing circuitry 730 can be one or more processing units (e.g., central processing units), microprocessors, microcontrollers (e.g., microcontroller units (MCUs)) or any other computing devices or modules, including multiple and/or parallel and/or distributed processing units, which are adapted to independently or cooperatively process data for controlling relevant greenhouse gas emission estimation and reduction system 700 resources and for enabling operations related to greenhouse gas emission estimation and reduction system's 700 resources.

Processing circuitry 730 can comprise a greenhouse gas emission estimation and reduction module 740. Greenhouse gas emission estimation and reduction module 740, can be configured to estimate, and facilitate reduction, of greenhouse gas emission, as further detailed herein, inter alia with reference to Fig. 11.

Turning to **Fig. 11****,** there is shown a flowchart illustrating one example of a sequence of operations carried out for estimating greenhouse gas emission in an environment housing an animal population, in accordance with the presently disclosed subject matter.

In accordance with the presently disclosed subject matter, greenhouse gas emission estimation and reduction system 700 can be configured to perform a greenhouse gas emission estimation and reduction process 800 (optionally in real-time or near-real time and optionally in a continuous or near-continuous manner), e.g., using greenhouse gas emission estimation and reduction module 740.

As indicated herein, data gathered from the monitoring devices is stored in records within a data repository. Each record is associated with a respective member of the animal population and holds descriptive data (e.g., age of the respective member, sex of the respective member, treatment history of the respective member, genetic information associated with the respective member, etc.) and monitoring data, e.g., parameters of the animal (for example: temperature, movement time, feeding time, social behavior time, respiration levels, rumination time, etc.) obtained from the monitoring devices. Monitoring data may be collected continuously, or near continuously, or periodically, or in some combination (where some data is collected periodically and some data is collected continuously or near continuously).

Greenhouse gas emission estimation and reduction system 700 is configured to obtain at least a subset of the records, the subset being associated with a group of members of the animal population (block 810). In some cases, all of the records of the animal population are obtained by greenhouse gas emission estimation and reduction system 700. In other cases, the group of members can consist of a sub-population of the animal population, selected in accordance with one or more criteria, such as a location of the sub-population. For example: the sub-population can be the animals that are located within a given farm. It is to be noted that the animal population can be located in one or more geographical sites and/or within specific locations within these sites (e.g., enclosures, pasture, treatment areas, etc.).

The animals of the animal population can be for example: ruminating animals, livestock, swine, companion animals or any other type of non-human animal.

After obtaining the at least subset of the records, greenhouse gas emission estimation and reduction system 700 is further configured to determine one or more greenhouse gas emission affecting parameters based on the subset (block 820). The greenhouse gas emission affecting parameters can include one or more of the following parameters:
(a) Heat Detection Rate (HDR) calculated for the group of members, noting that HDR can be measured by dividing a length of a predetermined heat cycle (e.g. 21 days for dairy cows) with an actual average length of estrus of the animals of the animal population (noting that heat can be detected based on the monitoring data analysis, as discussed, for example by: (a) A.M.L. Madureira, B.F. Silper, T.A. Burnett, L. Polsky, L.H. Cruppe, D.M. Veira, J.L.M. Vasconcelos, R.L.A. Cerri, "Factors affecting expression of estrus measured by activity monitors and conception risk of lactating dairy cows", Journal of Dairy Science, Volume 98, Issue 10,2015, Pages 7003-7014, (b) R.C. Neves, K.E. Leslie, J.S. Walton, S.J. LeBlanc, "Reproductive performance with an automated activity monitoring system versus a synchronized breeding program", Journal of Dairy Science, Volume 95, Issue 10, 2012, Pages 5683-5693, (c) S. Reith, S. Hoy, "Relationship between daily rumination time and estrus of dairy cows", Journal of Dairy Science, Volume 95, Issue 11, 2012, Pages 6416-6420, (d) S. Reith, H. Brandt, S. Hoy, "Simultaneous analysis of activity and rumination time, based on collar-mounted sensor technology, of dairy cows over the peri-estrus period", Livestock Science, Volume 170, 2014, Pages 219-227, and (e) S.P.M. Aungier, J.F. Roche, M. Sheehy, M.A. Crowe, "Effects of management and health on the use of activity monitoring for estrus detection in dairy cows", Journal of Dairy Science, Volume 95, Issue 5, 2012, Pages 2452-2466)
(b) a conception rate calculated for the group of members, indicative of a ratio of conceptions to estruses of animals of the group.
(c) a health score calculated for the group of members, that can be calculated as detailed herein, inter alia with reference to Figs. 1-3;
(d) rumination consistency, (also referred to herein as consistency of Rumination Variability (RV)), calculated for the group of members, based on the monitoring data; Rumination consistency is indicative of a level of consistency (e.g., on a scale of 1-100) of rumination time of the members of the group; In a certain example (non-limiting), the RV can be calculated by determining daily rumination averages of all members of the group (noting that rumination time of each member can be determined using the monitoring data, as known in the art) for a sliding window of 10 days, and then calculating a variability between the calculated averages calculated in the 10 days sliding window;
(e) rumination time heterogeneity calculated for the group of members, based on the monitoring data; Rumination time heterogeneity is indicative of the heterogeneity of rumination time of the members of the group; In a certain example, the rumination time heterogeneity can be calculated by determining a standard deviation between daily rumination times of the members of the animal population (noting that rumination time of each member can be determined using the monitoring data, as known in the art); or
(f) average grazing time calculated for the group of members during a given time period, based on the monitoring data; It is to be noted that grazing farms in which animals are fed mainly by grazing in pasture, are less effective in terms of milk production when compared to farms in which the animals are mainly fed with food compounds that are designed to increase the animals milk yield, and thus the GHG emission in such farms is not optimal as shifting to such compounds will result in less GHG emission per milk output for the same animal population. It is to be noted that a certain amount of grazing may not have a negative effect on the animal's milk yield (in some cases, even up to 50% of the feeding time spent grazing may not have a negative effect on milk yield).

When looking at HDR, some farms demonstrate an HDR of less than 60%. When compared with such farms, farms that demonstrate an HDR of 60%-70% demonstrate an improvement of about 10% in methane output (about 10% decrease in methane output per milk unit). Farms that demonstrate an HDR higher than 70% demonstrate an additional improvement of about 10% in methane output (and 20% improvement when compared with farms that demonstrate an HDR of less than 60%). Similarly, when looking at the conception rate, the higher the conception rate is, the lower the methane output is per milk unit.

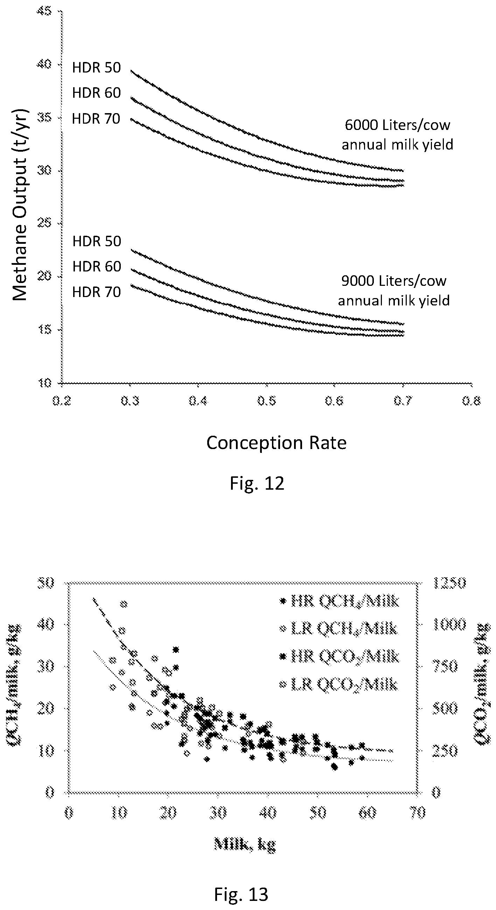

In the context of the HDR and conception rate, attention is drawn to Fig. 12. As can be seen when looking at Fig. 12, there is a connection between HDR and conception rates within an animal population on the one hand and methane and ammonia output produced by that animal population on the other hand, so that the higher the estrus detection and conception rates are - the lower the methane output per milk unit is. In addition, it can be appreciated from this figure that higher milk yielding cows generate less GHG per milk unit when compared to lower milk yielding cows. The graph shown in Fig. 12, which illustrates the connection between HDR and conception rates of an animal population on the one hand and methane and ammonia output produced by the animal population on the other hand, is taken from P.C Garnsworthy, "The environmental impact of fertility in dairy cows: a modelling approach to predict methane and ammonia emissions", Animal Feed Science and Technology, Volume 112, Issues 1-4, 2004, Pages 211-223.

When looking at the health score, it has been shown that Sub Clinical Ketosis (SCK) causes a direct milk loss of at least 5% of the milk production of dairy cows suffering from SCK. Some studies even demonstrate a direct milk loss of 7% of the milk production of dairy cows suffering from SCK (see P.F. Mostert, C.E. van Middelaar, E.A.M. Bokkers, I.J.M. de Boer, "The impact of subclinical ketosis in dairy cows on greenhouse gas emissions of milk production", Journal of Cleaner Production, Volume 171, 2018, Pages 773-782). Current systems are able to identify about 90% of the SCK cases. Thus, even if we assume that a direct milk loss of the milk production of dairy cows suffering from SCK is only 5%, and only 4% will be saved (and not all 5%), the savings in terms of GHG emissions are considerable (due to the fact that milk yield loss is avoided, and thus the GHG emission per unit of milk produced is reduced).

When looking at rumination consistency, it has been shown that rumination is highly correlated with fiber feed component particle length and quality (see, for example, (a) R.E. Coon, T.F. Duffield, T.J. DeVries, "Effect of straw particle size on the behavior, health, and production of early-lactation dairy cows", Journal of Dairy Science, Volume 101, Issue 7, 2018, Pages 6375-6387, (b) Coon RE, Duffield TF, DeVries TJ., "Short communication: Risk of subacute ruminal acidosis affects the feed sorting behavior and milk production of early lactation cows", Journal of Dairy Science, Volume 102, Issue 1, 2019, pages 652-659, and (c) G. Adin, R. Solomon, M. Nikbachat, A. Zenou, E. Yosef, A. Brosh, A. Shabtay, S.J. Mabjeesh, I. Halachmi, J. Miron, "Effect of feeding cows in early lactation with diets differing in roughage-neutral detergent fiber content on intake behavior, rumination, and milk production", Journal of Dairy Science, Volume 92, Issue 7, 2009, Pages 3364-3373). It has also been shown that inconsistency in fiber availability in the animal's feed can contribute to 3% reduction in milk production in a population of dairy cows (see A.D. Sova, S.J. LeBlanc, B.W. McBride, T.J. DeVries, "Accuracy and precision of total mixed rations fed on commercial dairy farms", Journal of Dairy Science, Volume 97, Issue 1, 2014, Pages 562-571).

Some farms demonstrate relatively poor rumination consistency in which rumination changes by over 20 minutes per day on average. Other farms demonstrate a rumination consistency between 10-20 minutes per day on average. Other farms demonstrate a rumination consistency of less than 10 minutes per day on average. In view of the fact that lower rumination consistency is attributable to feeding deficiency, and thus to lower milk yield (as detailed herein above), it is expected, in terms of CO₂ output, that (a) farms that demonstrate a rumination consistency between 10-20 minutes per day on average will demonstrate a decrease of 2% in CO₂ output per milk unit when compared with farms that demonstrate rumination consistency in which rumination changes by over 20 minutes per day on average, and (b) farms that demonstrate a rumination consistency of less than 10 minutes per day on average will demonstrate a decrease of 3% in CO₂ output per milk unit when compared with farms that demonstrate rumination consistency in which rumination changes by over 20 minutes per day on average.

When looking at rumination heterogeneity, assuming an expected rumination variability is 16%, as is the case when dealing with dairy cows (as shown in K.A. Beauchemin, "Invited review: Current perspectives on eating and rumination activity in dairy cows", Journal of Dairy Science, Volume 101, Issue 6, 2018, Pages 4762-4784), which translates to 80 minutes per day (based on 500 rumination minutes per day, according to the calculation of 500 * 0.16 = 80 minutes per day), some farms demonstrate relatively poor rumination heterogeneity in which the standard deviation of rumination between the animals is higher than 100 minutes per day. Other farms demonstrate a rumination heterogeneity in which the standard deviation of rumination between the animals is between 80-100 minutes per day. Other farms demonstrate a rumination heterogeneity in which the standard deviation of rumination between the animals is less than 80 minutes per day.

In terms of CO₂ output, farms that demonstrate a rumination heterogeneity in which the standard deviation of rumination between the animals is between 80-100 minutes per day demonstrate a decrease of 2% when compared with farms that demonstrate a standard deviation of rumination between the animals is higher than 100 minutes per day. Farms that demonstrate a rumination heterogeneity in which the standard deviation of rumination between the animals is less than 80 minutes per day demonstrate a decrease of 3% when compared with farms that demonstrate a standard deviation of rumination between the animals is higher than 100 minutes per day.

In this context, attention is drawn to Fig. 13, a schematic illustration of a relationship between yield of methane and carbon dioxide per units of milk, taken from an Watt et al.'s article "Differential rumination, intake, and enteric methane production of dairy cows in a pasture-based automatic milking system", published on Journal of Dairy Science, Volume 98, Issue 10, October 2015, Pages 7248-7263. In the graph, the relationship between yield of methane (QCH4/milk; circles) and carbon dioxide (*Q*CO2/milk; squares) per units of milk and the milk production of a population of dairy cows on a pasture-based automatic milking system is shown. Values for dairy cows classified as High Ruminating (HR, shown in solid symbols within the graph) or Low Ruminating (LR, shown in open symbols within the graph) are shown with corresponding exponential functions showing reductions of QCH4/milk (in the solid line) and of QCO2/milk (in the dashed line) as a function of milk production, across the animal population. Looking at the graph, it can be appreciated that there is a connection between the rumination heterogeneity and the methane and carbon dioxide emissions per milk unit, as low ruminating animals produce, on average, less milk (and thus more methane and carbon dioxide per milk unit), than high ruminating animals. The graph further indicates that the higher the milk yield of the animals is, the lower the methane and carbon dioxide emissions per milk unit are.

It is to be noted that these are exemplary parameters only, and in some cases, additional and/or alternative greenhouse gas emission affecting parameters can be calculated/obtained and used. In some cases, such additional and/or alternative greenhouse gas emission affecting parameters may include environmental parameters such as an ambient temperature in the environment of the animal population (as excessive heat in the animal population's environment can have a negative effect on the animal's milk yield (thus resulting in higher GHG emission per milk unit), etc. It is to be noted that every parameter that has an effect on milk yield of a given animal population can be taken into account, as an increase of milk yield of the animal population is likely to lead to an improved GHG emission per milk unit ratio.

Greenhouse gas emission estimation and reduction system 700 is configured to utilize the greenhouse gas emission affecting parameters in order to estimate an amount of greenhouse gas emission in the environment housing the animal population (block 830).

In some cases, the estimation can be based on comparison of the greenhouse gas emission affecting parameters to a given baseline. The given baseline can optionally be determined using direct measurement of greenhouse gas emission via greenhouse gas emission measuring equipment, acquired from the environment housing the animal population, or from another environment having known properties (e.g. size, size of animal population, or any other property that can be used to adapt the baseline to the environment housing the animal population). In some cases, the baseline can be geographical location specific (e.g., as geographical characteristics such as weather can have an effect on greenhouse gas emissions). In some cases, the baseline is determined based on all, or some, of the historical values of the greenhouse gas emission affecting parameters determined at block 820. It is to be noted that in such cases, where the baseline is determined based on historical values of greenhouse gas emission affecting parameters, the baseline can be validated using direct measurement of greenhouse gas emission acquired using greenhouse gas emission measuring equipment, acquired from the environment housing the animal population. Such validation can be a one-time validation occurring at a certain point in time, or a periodical validation occurring once in a while (e.g., once a month, once a year, once every few years, or any other time interval, which is not necessarily constant between subsequent validations).

Greenhouse gas emission estimation and reduction system 700 is further configured to perform an action utilizing the estimated amount of greenhouse gas emission in the environment (block 840). In some cases, the action can be one or more of: (a) providing a user of the system with an indication of the estimated amount of greenhouse gas emission, or (b) suggesting one or more greenhouse gas emission reduction actions to be taken on the animal population, or part/s thereof, in order to reduce the greenhouse gas emission in the environment. The greenhouse gas emission reduction actions can include one or more of: (i) administering a treatment to the animal population, or part/s thereof, (ii) changing a temperature of an environment of the animal population, or part/s thereof, (iii) changing a feed of the animal population, or part/s thereof (e.g. in order to improve the rumination consistency), (iv) changing a schedule of the animal population, or part/s thereof, (v) improving the reproduction of the animal population (e.g. improving the HDR, the conception rate, etc.), (vi) improving the welfare of the animal population, (vii) improving husbandry conditions, etc.

In some case, although not shown in the figure, following acting upon the recommendations, or performing any other action that the user deems fit, the user can update the greenhouse gas emission estimation and reduction system 700 with an indication of the actions taken. In such cases, the greenhouse gas emission estimation and reduction system 700 can be configured to repeat the greenhouse gas emission estimation and reduction process 800 following collection of additional data from the monitoring devices in order to estimate the effect of the actions made by the user on the GHG emission of the animal population. In this manner, the system can be used to evaluate the impact of various actions taken on GHG emissions.

It is to be noted that having the ability to provide a user of the system (e.g. a farmer) with an indication of the estimated amount of greenhouse gas emission generated by the animal population (e.g. the animals in the farmers farm) in real-time or near real-time is extremely advantageous, as it can enable the user to take action when there is a need. Automatic estimation of the greenhouse gas emission, using the teachings herein, can be made without expensive and hard to operate equipment, and optionally in real time or near real-time, as opposed to existing solutions which require human auditing or direct measurements acquired by dedicated instrumentation.

As indicated herein, human auditors base their GHG emission estimation on data collected in a slow and manual process that is prone to errors. As part of such process, the human auditors visit and visually inspect the facilities housing the animals, and gather various types of data on the animal population itself. Based at least in part on the human auditor's subjective impression, the human auditor estimates the GHG emissions in the audited environment. One exemplary problem of subjectivity of an auditor is its first impression of an animal facility. For a human auditor, it may be hard to shake off a first impression, or a previous impression (not necessarily first), of a facility. Such first impression can lead to erroneous assessment of GHG emissions in such facility. For example, if the animal facility makes a good first impression, the auditor may miss out on other problems, or give them lesser weight, or vice versa. It can be appreciated that efforts are made to train auditors to maintain standard levels of evaluation, however interobserver reliability can be low despite training of the human auditors. Even in those cases where auditors undergo identical training, that training needs to be continuous to hold high standards of consistency, and even in these cases, the subjectiveness bias is not, and cannot, be completely irradicated.

Thus, current GHG emission estimation methods are prone to the subjectivity of the auditor. On top of that, they are also sensitive to the data collection method, sensitive to the timing circumstances, and frequency of the inspection (lighting conditions, weather conditions, etc.), and require long gathering and processing times.

In addition, GHG emission estimations may vary between auditors, and between audits, because they are not produced in real-time, nor are they based on continuous monitoring. Human auditing is not based on continuous, or near continuous, monitoring of various parameters (e.g., parameters relating to the animals' environment, the animals' health measures, animal treatments, the weather, etc.), inter alia because it is impossible for a human to track such amounts of data, let alone track such amounts of data in a manner that will enable evaluating GHG emissions in the required speed. To the contrary, having an automatic mechanism for estimating GHG emissions, optionally in real-time or near real-time, continuously or near continuously, can enable a much completer and more accurate GHG emission estimation that is not dependent on outdated or no-longer relevant data.

As mentioned above, GHG emissions can be measured directly using dedicated instrumentation, however such instrumentation is usually unavailable for livestock growers to continuously and directly measure greenhouse gas emissions in the environment in which their livestock is grown. Direct measurement of GHG emissions is also an extremely difficult task to perform when the animals are not within a housing in which the dedicated instrumentation can yield accurate results. For example, dedicated instrumentation cannot provide an accurate measurement when the animal population is grazing in pasture because the actual gas emission generated by the animal population cannot be captured in an open area such as this (e.g. as the natural emissions generated by animals are below the sensitivity level of standard GHG measurement equipment).

Further, the system and method disclosed herein can provide the animal caregiver and/or a regulator (such as a governmental organization) with information that can be used to take action in real or near real time to improve greenhouse gas emissions, such as by obtaining treatments for veterinary or health issues such as: infectious diseases, ectoparasites, and/or reproductive issues.

Moreover, based on the system and method disclosed herein, benchmarks can be determined for one or more locations or facilities of animal populations. These benchmarks can be used as references over time to automatically determine the adherence of the facilities or locations with an expected level of greenhouse gas emissions. These benchmarks can be measured and validated, or adjusted if needed, optionally continuously.

The greenhouse gas emission estimation disclosed herein can be used to set standards (optionally industry wide standards), benchmark between different animal populations and/or between groups of members of one or more animal populations, and provide management or veterinarian support, optionally in real time, to reduce greenhouse gas emissions. The greenhouse gas emission estimation disclosed herein can also be used by insurance and/or finance providers to evaluate risks in insuring and/or financing related to the animal population.

For example, the greenhouse gas emission estimation disclosed herein can be used to set standards for greenhouse gas emissions for various types of animal caregivers, whether institutional (such as farms in which cows, swine, equine or any other type of animal is maintained, for slaughter, milk production or for any other purpose) or private animal owners (such as pet owners). Having such standards can enable regulation (enabling governments or other regulators make sure that greenhouse gas emission meets the required standards, and quickly identify any irregularity), value and/or risk estimation (enabling insurers, lenders or other type of financial institutions, trade associations, customers, or any other entity that is interested in valuation and/or risk estimation of the animals or their products, to more accurately evaluate the animals or their products such as milk, meat, etc.), and more. For example, the improved objectivity, consistency, frequency, and reliability of the greenhouse gas emission estimation and reduction systems and methods descried herein allows for more accurate and precise comparison between disparate farms/animal facilities, which is one of the advantages that enables entities to generate effective standard setting, risk estimation regulation, etc.

In some cases, the greenhouse gas emission estimation can also be used as a measure based on which a certification of greenhouse gas emissions can be provided, similarly to the Marine Stewardship Council (MSC) Certification used in the fishing industry.

It is to be noted that the ability of the system disclosed herein to monitor the greenhouse gas emission estimation associated with animal populations in a continuous (or near continuous) reliable, consistent, and objective manner, is extremely powerful and important. The monitoring described herein enables all stakeholders (animal caregivers, regulators, financial institutions, trade associations, milk processors, meat processors, retail chains, end-customers, or any other entity that may have an interest in the animals' welfare) to receive relevant information for making decisions in real-time, or almost in real-time (e.g., within milliseconds, seconds, minutes, hours or days) based on continuously, or near continuously, updated reliable, consistent, and objective information (as opposed to existing solutions in which such information is unavailable and requires transferring expensive and hard to operate greenhouse gas emission measurement equipment).

A few non-limiting examples for uses of the greenhouse gas emission estimation according to the presently disclosed subject matter: financial institutions can evaluate risks based on relevant information indicative of the greenhouse gas emission estimation at the time they make decisions; Regulators can act upon a detected irregularity when it occurs and not in retrospect; retail chains, milk processors, meat processors, end-customers, or any other consumers of products of the animal population can get information of the greenhouse gas emission estimation of the animal population from which the products originate; diary and/or meat producers can compare farms based on the greenhouse gas emission estimation, so they can make informed purchasing decisions.

It is important to note that the greenhouse gas emission estimation and reduction according to the teachings herein is determined based, at least in part, on direct analysis of the animals behavioural and physiological signals and not based on human interpretation or measurement by dedicated greenhouse gas emission measurement machinery.

The greenhouse gas emission estimation can be also used by regulation entities or sourcing companies for monitoring regulated or supplier farms, develop procedures with the animal care giver they regulate or source from, manage public and customer opinion and ensure they maintain transparency around their brand name and brand value.

It is to be noted, with reference to Fig. 11, that some of the blocks can be integrated into a consolidated block or can be broken down to a few blocks and/or other blocks may be added. It should be also noted that whilst the flow diagram is described also with reference to the system elements that realizes them, this is by no means binding, and the blocks can be performed by elements other than those described herein.

It is to be understood that the presently disclosed subject matter is not limited in its application to the details set forth in the description contained herein or illustrated in the drawings. The presently disclosed subject matter is capable of other embodiments and of being practiced and carried out in various ways. Hence, it is to be understood that the phraseology and terminology employed herein are for the purpose of description and should not be regarded as limiting. As such, those skilled in the art will appreciate that the conception upon which this disclosure is based may readily be utilized as a basis for designing other structures, methods, and systems for carrying out the several purposes of the present presently disclosed subject matter.

It will also be understood that the system according to the presently disclosed subject matter can be implemented, at least partly, as a suitably programmed computer. Likewise, the presently disclosed subject matter contemplates a computer program being readable by a computer for executing the disclosed methods. The presently disclosed subject matter further contemplates a machine-readable memory tangibly embodying a program of instructions executable by the machine for executing the disclosed methods.

## Claims

1. A system for estimating greenhouse gas emission in an environment housing an animal population, the system comprising:
one or more monitoring devices configured to monitor parameters of members of the animal population;
a data repository (710) comprising two or more records, each of the records (i) being associated with a respective member of the members, and (ii) including one or more monitored parameters of the respective member as monitored by at least one of the monitoring devices over time; and
a processing circuitry (730) configured to:
obtain at least a subset of the records, the subset being associated with a group of given members of the members;
determine one or more greenhouse gas emission affecting parameters based on the subset, wherein at least one greenhouse gas emission affecting parameter of the greenhouse gas emission affecting parameters is determined based on one or more of the monitored parameters that are monitored across at least two members of the group of given members;
estimate an amount of greenhouse gas emission in the environment utilizing the greenhouse gas emission affecting parameters based at least in part on a comparison of at least one greenhouse gas emission affecting parameter with at least one baseline determined using greenhouse gas emission measurements acquired from the environment housing the animal population using greenhouse gas emission measuring equipment; and
suggest an action utilizing the estimated amount of greenhouse gas emission in the environment.

2. The system according to claim 1, wherein the greenhouse gas emission affecting parameters include one or more of the following: (a) a welfare score indicative of a health state of the group of given members, (b) Heat Detection Rate (HDR) calculated for the group of given members, (c) a conception rate calculated for the group of given members, (d) a health score calculated for the group of given members, (e) rumination consistency calculated for the group of given members, or (f) rumination time heterogeneity calculated for the group of given members.

3. The system of claim 2, wherein the processing circuitry (730) is further configured to calculate, based on the subset of the records, for each given member of the group of given members, at least one of: (a) an animal health score indicative of a health state of the respective member, (b) an animal natural living score, indicative of compliance of a behavior pattern of the respective member with a desired natural behavior pattern, or (c) an animal affectivity/happiness score, indicative of compliance of an affectivity/happiness measure of the respective member with a desired affectivity/happiness measure, the affectivity/happiness measure being determined based on one or more of the following monitored parameters, being affectivity/happiness parameters: (1) respiration level of the given member, (2) percentage of rumination time within a fourth time period of the given member, or (3) percentage of feeding time within a fifth time period of the given member; and
wherein:
(i) the health score is calculated based on the animal health scores calculated for the group of given members; and
(ii) the welfare score is calculated based on the at least one of: (a) the health score, (b) the animal natural living scores calculated for the group of given members, or (c) the animal affectivity/happiness scores calculated for the group of given members.

4. A method for estimating greenhouse gas emission in an environment housing an animal population, the method comprising:
obtaining, by a processing circuitry (730), at least a subset of two or more records, each of the records (i) being associated with a respective member of members of the animal population, and (ii) including one or more monitored parameters of the respective member as monitored over time by at least one of a group of one or more monitoring devices configured to monitor parameters of the members of the animal population, wherein the subset is associated with a first group of given members of the members;
determining, by the processing circuitry (730), one or more greenhouse gas emission affecting parameters based on the subset, wherein at least one greenhouse gas emission affecting parameter of the greenhouse gas emission affecting parameters is determined based on one or more of the monitored parameters that are monitored across at least two members of the first group of given members;
estimating, by the processing circuitry (730), an amount of greenhouse gas emission in the environment utilizing the greenhouse gas emission affecting parameters based at least in part on a comparison of at least one greenhouse gas emission affecting parameter with at least one baseline determined using greenhouse gas emission measurements acquired from the environment housing the animal population using greenhouse gas emission measuring equipment; and suggesting, by the processing circuitry (730), an action utilizing the estimated amount of greenhouse gas emission in the environment.

5. The system according to claim 1, or the method according to claim 4, wherein the baseline is determined based on historical values of the greenhouse gas emission affecting parameters.

6. The system according to any one of claims 1-3, or the method according to any one of claims 4 to 5 , wherein the action is one or more of: (a) providing a user with an indication of the estimated amount of greenhouse gas emission, or (b) suggesting one or more greenhouse gas emission reduction actions to be taken on at least part of the animal population in order to reduce the greenhouse gas emission in the environment.

7. The system according to claim 6, or the method according to claim 6, wherein the greenhouse gas emission reduction actions include one or more of: administering a treatment to the at least part of the animal population, changing a temperature of an environment of the at least part of the animal population, changing a feed of the at least part of the animal population, or changing a schedule of the at least part of the animal population.

8. The method according to any one of claims 4 to 7, wherein the greenhouse gas emission affecting parameters include one or more of the following: (a) a welfare score indicative of a health state of the first group of given members, (b) Heat Detection Rate (HDR) calculated for the first group of given members, (c) a conception rate calculated for the first group of given members, (d) a health score calculated for the first group of given members, (e) rumination consistency calculated for the first group of given members, or (f) rumination time heterogeneity calculated for the first group of given members.

9. The method of claim 8, further comprising calculating, based on the subset of the records, for each given member of the first group of given members, at least one of: (a) an animal health score indicative of a health state of the respective member, (b) an animal natural living score, indicative of compliance of a behavior pattern of the respective member with a desired natural behavior pattern, or (c) an animal affectivity/happiness score, indicative of compliance of an affectivity/happiness measure of the respective member with a desired affectivity/happiness measure, the affectivity/happiness measure being determined based on one or more of the following monitored parameters, being affectivity/happiness parameters: (1) respiration level of the given member, (2) percentage of rumination time within a fourth time period of the given member, or (3) percentage of feeding time within a fifth time period of the given member; and
wherein:
(iii) the health score is calculated based on the animal health scores calculated for the first group of given members; and
(iv) the welfare score is calculated based on the at least one of: (a) the health score, (b) the animal natural living scores calculated for the first group of given members, or (c) the animal affectivity/happiness scores calculated for the first group of given members.

10. The system according to claim 3, or the method according to claim 9, wherein the monitored parameters include one or more of the following behavioral parameters: (a) percentage of movement time within a first time period of the given member, (b) percentage of feeding time within a second time period of the given member or (c) percentage of social behavior time within a third time period of the given member; and wherein the animal natural living score for the given member is determined based on the behavioral parameters.

11. The system according to claim 10, or the method according to claim 10, wherein the natural living score for the given member is determined based on consistency of values of at least some of the behavioral parameters over time.

12. The system of claim 3, or the method according to claim 9, wherein the affectivity/happiness score for the given member is determined based on consistency of values of at least some of the affectivity/happiness parameters over time.

13. The system according to claim 2 or 3, or the method according to any one of claims 8 or 9, wherein the welfare score is calculated based on a variation between the affectivity/happiness scores of the first group of given members.

14. The system of claim 3, or the method according to claim 9 wherein the records also include one or more environmental parameters, indicative of the state of the environment of the respective member and wherein the determination of (a) the animal natural living score of the respective member or (b) of the animal affectivity/happiness score of the respective member, is also based on the environmental parameters.

15. A non-transitory computer readable storage medium having computer readable program code embodied therewith, the computer readable program code, executable by at least one processing circuitry (730) of a computer to perform a method for estimating greenhouse gas emission in an environment housing an animal population, the method comprising:
obtaining, by the processing circuitry (730), at least a subset of two or more records, each of the records (i) being associated with a respective member of members of the animal population, and (ii) including one or more monitored parameters of the respective member as monitored over time by at least one of a group of one or more monitoring devices configured to monitor parameters of the members of the animal population, wherein the subset is associated with a first group of given members of the members;
determining, by the processing circuitry (730), one or more greenhouse gas emission affecting parameters based on the subset, wherein at least one greenhouse gas emission affecting parameter of the greenhouse gas emission affecting parameters is determined based on one or more of the monitored parameters that are monitored across at least two members of the first group of given members;
estimating, by the processing circuitry (730), an amount of greenhouse gas emission in the environment utilizing the greenhouse gas emission affecting parameters based at least in part on a comparison of at least one greenhouse gas emission affecting parameter with at least one baseline determined using greenhouse gas emission measurements acquired from the environment housing the animal population using greenhouse gas emission measuring equipment; and suggesting, by the processing circuitry (730), an action utilizing the estimated amount of greenhouse gas emission in the environment.

## Patentansprüche

1. System zum Schätzen von Treibhausgasemissionen in einer Umgebung, in der sich eine Tierpopulation befindet, wobei das System umfasst:
eine oder mehrere Überwachungsvorrichtungen, die dazu ausgelegt sind, Parameter von Mitgliedern der Tierpopulation zu überwachen;
einen Daten-Repository (710), der zwei oder mehr Datensätze umfasst, wobei jeder der Datensätze (i) mit einem jeweiligen Mitglied der Mitglieder assoziiert ist und (ii) einen oder mehrere überwachte Parameter des jeweiligen Mitglieds einschließt, wie sie durch mindestens eine der Überwachungsvorrichtungen im Laufe der Zeit überwacht werden; und
eine Verarbeitungsschaltungsanordnung (730), die ausgelegt ist zum:
Erhalten mindestens einer Teilmenge der Datensätze, wobei die Teilmenge mit einer Gruppe von gegebenen Mitgliedern der Mitglieder assoziiert ist;
Bestimmen eines oder mehrerer Treibhausgasemissionen beeinflussender Parameter basierend auf der Teilmenge, wobei mindestens ein Treibhausgasemissionen beeinflussender Parameter der Treibhausgasemissionen beeinflussenden Parameter basierend auf einem oder mehreren der überwachten Parameter bestimmt wird, die über mindestens zwei Mitglieder der Gruppe von gegebenen Mitgliedern überwacht werden;
Schätzen einer Menge von Treibhausgasemissionen in der Umwelt unter Nutzung der Treibhausgasemissionen beeinflussenden Parameter basierend zumindest teilweise auf einem Vergleich von zumindest einem Treibhausgasemissionen beeinflussenden Parameter mit zumindest einer Basislinie, die unter Verwendung von Treibhausgasemissionsmessungen bestimmt wird, die von der Umgebung, in der sich die Tierpopulation befindet, unter Verwendung von Treibhausgasemissionsmessgeräten erfasst wird; und
Vorschlagen einer Aktion unter Nutzung der geschätzten Menge an Treibhausgasemissionen in der Umwelt.

2. System nach Anspruch 1, wobei die Treibhausgasemissionen beeinflussenden Parameter eines oder mehrere der Folgenden einschließen: (a) einen Wohlbefindensscore, der einen Gesundheitszustand der Gruppe gegebener Mitglieder angibt, (b) eine Wärmedetektionsrate (HDR), die für die Gruppe gegebener Mitglieder berechnet wird, (c) eine Konzeptionsrate, die für die Gruppe gegebener Mitglieder berechnet wird, (d) einen Gesundheitsscore, der für die Gruppe gegebener Mitglieder berechnet wird, (e) Wiederkäukonsistenz, die für die Gruppe gegebener Mitglieder berechnet wird, oder (f) Heterogenität der Wiederkäuzeit, die für die Gruppe gegebener Mitglieder berechnet wird.

3. System nach Anspruch 2, wobei die Verarbeitungsschaltungsanordnung (730) ferner dazu ausgelegt ist, basierend auf der Teilmenge der Datensätze für jedes gegebene Mitglied der Gruppe von gegebenen Mitgliedern mindestens eines von Folgendem zu berechnen: (a) einem Tiergesundheitsscore, der einen Gesundheitszustand des jeweiligen Mitglieds angibt, (b) einem Score des natürlichen Lebens des Tieres, der eine Konformität eines Verhaltensmusters des jeweiligen Mitglieds mit einem gewünschten natürlichen Verhaltensmuster angibt, oder (c) einem Affektivitäts-/Glücklichkeitsscore des Tieres, der eine Konformität eines Affektivitäts-/Glücklichkeitsmesswerts des jeweiligen Mitglieds mit einem gewünschten Affektivitäts-/Glücklichkeitsmesswert angibt, wobei der Affektivitäts-/Glücklichkeitsmesswert basierend auf einem oder mehreren der folgenden überwachten Parameter bestimmt wird, die Affektivitäts-/Glücklichkeitsparameter sind: (1) Atemniveau des gegebenen Mitglieds, (2) Prozentsatz der Wiederkäuzeit innerhalb einer vierten Zeitperiode des gegebenen Mitglieds oder (3) Prozentsatz der Fresszeit innerhalb einer fünften Zeitperiode des gegebenen Mitglieds; und wobei:
(i) der Gesundheitsscore basierend auf den Tiergesundheitsscores berechnet wird, die für die Gruppe der gegebenen Mitglieder berechnet werden; und
(ii) der Wohlbefindensscore basierend auf mindestens einem der Folgenden berechnet wird: (a) dem Gesundheitsscore, (b) den Scores des natürlichen Lebens des Tieres, die für die Gruppe von gegebenen Mitgliedern berechnet werden, oder (c) den Affektivitäts-/Glücklichkeitsscores des Tieres, die für die Gruppe von gegebenen Mitgliedern berechnet werden.

4. Verfahren zum Schätzen von Treibhausgasemissionen in einer Umgebung, in der sich eine Tierpopulation befindet, wobei das Verfahren umfasst:
Erhalten mindestens einer Teilmenge von zwei oder mehr Datensätzen durch die Verarbeitungsschaltungsanordnung (730), wobei jeder der Datensätze (i) mit einem jeweiligen Mitglied von Mitgliedern der Tierpopulation assoziiert ist, und (ii) ein oder mehrere überwachte Parameter des jeweiligen Mitglieds einschließt, wie sie im Laufe der Zeit durch mindestens eine aus einer Gruppe von einer oder mehreren Überwachungsvorrichtungen überwacht werden, die dazu ausgelegt sind, Parameter der Mitglieder der Tierpopulation zu überwachen, wobei die Teilmenge mit einer ersten Gruppe von gegebenen Mitgliedern der Mitglieder assoziiert ist;
Bestimmen eines oder mehrerer Treibhausgasemissionen beeinflussender Parameter basierend auf der Teilmenge durch die Verarbeitungsschaltungsanordnung (730), wobei mindestens ein Treibhausgasemissionen beeinflussender Parameter der Treibhausgasemissionen beeinflussenden Parameter basierend auf einem oder mehreren der überwachten Parameter bestimmt wird, die über mindestens zwei Mitglieder der ersten Gruppe von gegebenen Mitgliedern überwacht werden;
Schätzen einer Menge an Treibhausgasemissionen in der Umgebung unter Nutzung der Treibhausgasemissionen beeinflussenden Parameter durch die Verarbeitungsschaltungsanordnung (730) mindestens teilweise basierend auf einem Vergleich mindestens eines Treibhausgasemissionen beeinflussenden Parameters mit mindestens einer Basislinie, die unter Verwendung von Treibhausgasemissionsmessungen bestimmt wird, die von der Umgebung, in der sich die Tierpopulation befindet, unter Verwendung von Treibhausgasemissionsmessgeräten erfasst werden; und
Vorschlagen einer Aktion unter Nutzung der geschätzten Menge an Treibhausgasemissionen in der Umgebung durch die Verarbeitungsschaltungsanordnung (730).

5. System nach Anspruch 1 oder Verfahren nach Anspruch 4, wobei die Basislinie basierend auf historischen Werten der Treibhausgasemissionen beeinflussenden Parameter bestimmt wird.

6. System nach einem der Ansprüche 1-3 oder Verfahren nach einem der Ansprüche 4 bis 5, wobei die Aktion eine oder mehrere von Folgendem ist: (a) Bereitstellen einer Angabe über die geschätzte Menge an Treibhausgasemissionen an einen Benutzer, oder (b) Vorschlagen von einer oder mehreren Aktionen zur Reduzierung der Treibhausgasemissionen, die an mindestens einem Teil der Tierpopulation durchzuführen sind, um die Treibhausgasemissionen in der Umwelt zu reduzieren.

7. System nach Anspruch 6 oder Verfahren nach Anspruch 6, wobei die Aktionen zur Reduzierung der Treibhausgasemissionen eines oder mehrere von Folgendem einschließen: Verabreichen einer Behandlung an den mindestens einen Teil der Tierpopulation, Ändern einer Temperatur einer Umgebung des mindestens einen Teils der Tierpopulation, Ändern eines Futtermittels des mindestens einen Teils der Tierpopulation oder Ändern eines Zeitplans des mindestens einen Teils der Tierpopulation.

8. Verfahren nach einem der Ansprüche 4 bis 7, wobei die Treibhausgasemissionen beeinflussenden Parameter eines oder mehrere der Folgenden einschließen: (a) einen Wohlbefindensscore, der einen Gesundheitszustand der ersten Gruppe gegebener Mitglieder angibt, (b) eine Wärmedetektionsrate (HDR), die für die erste Gruppe gegebener Mitglieder berechnet wird, (c) eine Konzeptionsrate, die für die erste Gruppe gegebener Mitglieder berechnet wird, (d) einen Gesundheitsscore, die für die erste Gruppe gegebener Mitglieder berechnet wird, (e) Wiederkäukonsistenz, die für die erste Gruppe gegebener Mitglieder berechnet wird, oder (f) Heterogenität der Wiederkäuzeit, die für die erste Gruppe gegebener Mitglieder berechnet wird.

9. Verfahren nach Anspruch 8, ferner umfassend Berechnen für jedes gegebene Mitglied der ersten Gruppe von gegebenen Mitgliedern von mindestens einem der Folgenden basierend auf der Teilmenge der Datensätze: (a) einem Tiergesundheitsscore, der einen Gesundheitszustand des jeweiligen Mitglieds angibt, (b) einem Score des natürlichen Lebens des Tieres, der eine Konformität eines Verhaltensmusters des jeweiligen Mitglieds mit einem gewünschten natürlichen Verhaltensmuster angibt, oder (c) einem Affektivitäts-/Glücklichkeitsscore des Tieres, der eine Konformität eines Affektivitäts-/Glücklichkeitsmesswerts des jeweiligen Mitglieds mit einem gewünschten Affektivitäts-/Glücklichkeitsmesswert angibt, wobei der Affektivitäts-/Glücklichkeitsmesswert basierend auf einem oder mehreren der folgenden überwachten Parameter bestimmt wird, die Affektivitäts-/Glücklichkeitsparameter sind: (1) Atemniveau des gegebenen Mitglieds, (2) Prozentsatz der Wiederkäuzeit innerhalb einer vierten Zeitperiode des gegebenen Mitglieds oder (3) Prozentsatz der Fresszeit innerhalb einer fünften Zeitperiode des gegebenen Mitglieds; und wobei:
(iii) der Gesundheitsscore basierend auf den Tiergesundheitsscores berechnet wird, die für die erste Gruppe bestimmter Mitglieder berechnet wurden; und
(iv) der Wohlbefindensscore basierend auf mindestens einem der Folgenden berechnet wird: (a) dem Gesundheitsscore, (b) den Scores des natürlichen Lebens des Tieres, die für die erste Gruppe von gegebenen Mitgliedern berechnet werden, oder (c) den Affektivitäts-/Glücklichkeitsscores des Tieres, die für die erste Gruppe von gegebenen Mitgliedern berechnet werden.

10. System nach Anspruch 3 oder Verfahren nach Anspruch 9, wobei die überwachten Parameter einen oder mehrere der folgenden Verhaltensparameter einschließen: (a) Prozentsatz der Bewegungszeit innerhalb einer ersten Zeitperiode des gegebenen Mitglieds, (b) Prozentsatz der Fresszeit innerhalb einer zweiten Zeitperiode des gegebenen Mitglieds oder (c) Prozentsatz der Sozialverhaltenszeit innerhalb einer dritten Zeitperiode des gegebenen Mitglieds; und wobei der Score für natürliches Leben des Tieres für das gegebene Mitglied basierend auf den Verhaltensparametern bestimmt wird.

11. System nach Anspruch 10 oder Verfahren nach Anspruch 10, wobei der Score für natürliches Leben für das gegebene Mitglied basierend auf einer Konsistenz von Werten von mindestens einigen der Verhaltensparameter über die Zeit bestimmt wird.

12. System nach Anspruch 3 oder Verfahren nach Anspruch 9, wobei der Affektivitäts-/Glücklichkeitsscore für das gegebene Mitglied basierend auf einer Konsistenz von Werten von mindestens einigen der Affektivitäts-/Glücklichkeitsparameter über die Zeit bestimmt wird.

13. System nach Anspruch 2 oder 3 oder Verfahren nach einem der Ansprüche 8 oder 9, wobei der Wohlbefindensscore basierend auf einer Variation zwischen den Affektivitäts-/Glücklichkeitsscores der ersten Gruppe von gegebenen Mitgliedern berechnet wird.

14. System nach Anspruch 3 oder Verfahren nach Anspruch 9, wobei die Datensätze auch einen oder mehrere Umgebungsparameter einschließen, die den Zustand der Umgebung des jeweiligen Mitglieds angeben, und wobei die Bestimmung von (a) dem Score für natürliches Leben des Tiers des jeweiligen Mitglieds oder (b) den Affektivitäts-/Glücklichkeitsscores des jeweiligen Mitglieds auch auf den Umgebungsparametern basiert.

15. Nichtflüchtiges computerlesbares Speichermedium mit damit verkörpertem computerlesbarem Programmcode, wobei der computerlesbare Programmcode durch mindestens eine Verarbeitungsschaltungsanordnung (730) eines Computers ausführbar ist, um ein Verfahren zum Schätzen von Treibhausgasemissionen in einer Umgebung durchzuführen, in der sich eine Tierpopulation befindet, wobei das Verfahren umfasst:
Erhalten mindestens einer Teilmenge von zwei oder mehr Datensätzen durch die Verarbeitungsschaltungsanordnung (730), wobei jeder der Datensätze (i) mit einem jeweiligen Mitglied von Mitgliedern der Tierpopulation assoziiert ist, und (ii) ein oder mehrere überwachte Parameter des jeweiligen Mitglieds einschließt, wie sie im Laufe der Zeit durch mindestens eine aus einer Gruppe von einer oder mehreren Überwachungsvorrichtungen überwacht werden, die dazu ausgelegt sind, Parameter der Mitglieder der Tierpopulation zu überwachen, wobei die Teilmenge mit einer ersten Gruppe von gegebenen Mitgliedern der Mitglieder assoziiert ist;
Bestimmen eines oder mehrerer Treibhausgasemissionen beeinflussender Parameter basierend auf der Teilmenge durch die Verarbeitungsschaltungsanordnung (730), wobei mindestens ein Treibhausgasemissionen beeinflussender Parameter der Treibhausgasemissionen beeinflussenden Parameter basierend auf einem oder mehreren der überwachten Parameter bestimmt wird, die über mindestens zwei Mitglieder der ersten Gruppe von gegebenen Mitgliedern überwacht werden;
Schätzen einer Menge an Treibhausgasemissionen in der Umgebung unter Nutzung der Treibhausgasemissionen beeinflussenden Parameter durch die Verarbeitungsschaltungsanordnung (730) mindestens teilweise basierend auf einem Vergleich mindestens eines Treibhausgasemissionen beeinflussenden Parameters mit mindestens einer Basislinie, die unter Verwendung von Treibhausgasemissionsmessungen bestimmt wird, die von der Umgebung, in der sich die Tierpopulation befindet, unter Verwendung von Treibhausgasemissionsmessgeräten erfasst werden; und
Vorschlagen einer Aktion unter Nutzung der geschätzten Menge an Treibhausgasemissionen in der Umgebung durch die Verarbeitungsschaltungsanordnung (730).

## Revendications

1. Système d'estimation d'émissions de gaz à effet de serre dans un environnement logeant une population animale, le système comprenant :
un ou plusieurs dispositifs de surveillance configurés pour surveiller des paramètres de membres de la population animale ;
un référentiel de données (710) comprenant deux enregistrements ou plus, chacun des enregistrements (i) étant associé à un membre respectif des membres, et (ii) incluant un ou plusieurs paramètres surveillés du membre respectif tel que surveillés dans le temps par au moins l'un des dispositifs de surveillance ; et
un circuit de traitement (730) configuré pour :
obtenir au moins un sous-ensemble des enregistrements, le sous-ensemble étant associé à un groupe de membres donnés des membres ;
déterminer un ou plusieurs paramètres affectant les émissions de gaz à effet de serre sur la base du sous-ensemble, au moins un paramètre affectant les émissions de gaz à effet de serre des paramètres affectant les émissions de gaz à effet de serre étant déterminé sur la base d'un ou de plusieurs des paramètres surveillés qui sont surveillés sur au moins deux membres du groupe de membres donnés ;
estimer une quantité d'émissions de gaz à effet de serre dans l'environnement en utilisant les paramètres affectant les émissions de gaz à effet de serre en se basant au moins en partie sur une comparaison d'au moins un paramètre affectant les émissions de gaz à effet de serre avec au moins un niveau de référence déterminé à l'aide de mesures d'émissions de gaz à effet de serre acquises dans l'environnement abritant la population animale au moyen d'un équipement de mesure d'émissions de gaz à effet de serre ; et
suggérer une action en utilisant la quantité estimée d'émissions de gaz à effet de serre dans l'environnement.

2. Système selon la revendication 1, dans lequel les paramètres affectant les émissions de gaz à effet de serre incluent un ou plusieurs des paramètres suivants : (a) un score de bien-être indicatif de l'état de santé du groupe de membres donnés, (b) un taux de détection de chaleur (HDR) calculé pour le groupe de membres donnés, (c) un taux de conception calculé pour le groupe de membres donnés, (d) un score de santé calculé pour le groupe de membres donnés, (e) une cohérence de rumination calculée pour le groupe de membres donnés, ou (f) une hétérogénéité de temps de rumination calculée pour le groupe de membres donnés.

3. Système selon la revendication 2, dans lequel le circuit de traitement (730) est en outre configuré pour calculer, sur la base du sous-ensemble des enregistrements, pour chaque membre donné du groupe de membres donnés, au moins l'un des éléments suivants : (a) un score de santé de l'animal indiquant un état de santé du membre respectif, (b) un score de vie naturelle de l'animal, indiquant la conformité d'un modèle de comportement du membre respectif avec un modèle de comportement naturel désiré, ou (c) un score d'affectivité/bonheur de l'animal, indiquant la conformité d'une mesure d'affectivité/bonheur du membre respectif avec une mesure d'affectivité/bonheur désirée, la mesure d'affectivité/bonheur étant déterminée sur la base d'un ou plusieurs des paramètres surveillés suivants, qui sont des paramètres d'affectivité/bonheur : (1) le niveau de respiration du membre donné, (2) le pourcentage du temps de rumination au cours d'une quatrième période de temps du membre donné, ou (3) le pourcentage du temps d'alimentation au cours d'une cinquième période de temps du membre donné ; et
dans lequel :
(i) le score de santé est calculé en fonction des scores de santé des animaux calculés pour le groupe de membres donnés ; et
(ii) le score de bien-être est calculé en fonction de l'un au moins des éléments suivants : (a) le score de santé, (b) les scores de vie naturelle des animaux calculés pour le groupe de membres donnés, ou (c) les scores d'affectivité/bonheur des animaux calculés pour le groupe de membres donnés.

4. Procédé d'estimation d'émissions de gaz à effet de serre dans un environnement logeant une population animale, le procédé comprenant :
l'obtention, par un circuit de traitement (730), d'au moins un sous-ensemble de deux enregistrements ou plus, chacun des enregistrements (i) étant associé à un membre respectif de membres de la population animale, et (ii) incluant un ou plusieurs paramètres surveillés du membre respectif tels que surveillés dans le temps par au moins l'un d'un groupe d'un ou plusieurs dispositifs de surveillance configurés pour surveiller des paramètres des membres de la population animale, le sous-ensemble étant associé à un premier groupe de membres donnés des membres ;
la détermination, par le circuit de traitement (730), d'un ou plusieurs paramètres affectant les émissions de gaz à effet de serre sur la base du sous-ensemble, au moins un paramètre affectant les émissions de gaz à effet de serre des paramètres affectant les émissions de gaz à effet de serre étant déterminé sur la base d'un ou de plusieurs des paramètres surveillés qui sont surveillés sur au moins deux membres du premier groupe de membres donnés ;
l'estimation, par le circuit de traitement (730), d'une quantité d'émissions de gaz à effet de serre dans l'environnement en utilisant les paramètres affectant les émissions de gaz à effet de serre en se basant au moins en partie sur une comparaison d'au moins un paramètre affectant les émissions de gaz à effet de serre avec au moins un niveau de référence déterminé à l'aide de mesures d'émissions de gaz à effet de serre acquises dans l'environnement abritant la population animale au moyen d'un équipement de mesure d'émissions de gaz à effet de serre ; et
la suggestion, par le circuit de traitement (730), d'une action en utilisant la quantité estimée d'émissions de gaz à effet de serre dans l'environnement.

5. Système selon la revendication 1, ou procédé selon la revendication 4, dans lequel la ligne de base est déterminée sur la base de valeurs historiques des paramètres affectant les émissions de gaz à effet de serre.

6. Système selon l'une quelconque des revendications 1 à 3, ou procédé selon l'une quelconque des revendications 4 à 5, dans lequel l'action est l'une ou plusieurs parmi : a) la fourniture à un utilisateur d'une indication de la quantité estimée d'émissions de gaz à effet de serre, ou b) la suggestion d'une ou plusieurs actions de réduction des émissions de gaz à effet de serre à prendre sur au moins une partie de la population animale afin de réduire les émissions de gaz à effet de serre dans l'environnement.

7. Système selon la revendication 6, ou procédé selon la revendication 6, dans lequel les actions de réduction des émissions de gaz à effet de serre comprennent l'une ou plusieurs parmi : l'administration d'un traitement à l'au moins une partie de la population animale, le changement d'une température d'un environnement de l'au moins une partie de la population animale, le changement d'une alimentation de l'au moins une partie de la population animale, ou le changement d'un programme de l'au moins une partie de la population animale.

8. Procédé selon l'une quelconque des revendications 4 à 7, dans lequel les paramètres affectant les émissions de gaz à effet de serre comprennent un ou plusieurs des suivants : (a) un score de bien-être indicatif de l'état de santé du premier groupe de membres donnés, (b) un taux de détection de chaleur (HDR) calculé pour le premier groupe de membres donnés, (c) un taux de conception calculé pour le premier groupe de membres donnés, (d) un score de santé calculé pour le premier groupe de membres donnés, (e) une cohérence de rumination calculée pour le premier groupe de membres donnés, ou (f) une hétérogénéité de temps de rumination calculée pour le premier groupe de membres donnés.

9. Procédé selon la revendication 8, comprenant en outre le calcul, sur la base du sous-ensemble des enregistrements, pour chaque membre donné du premier groupe de membres donnés, d'au moins l'un des éléments suivants : (a) un score de santé de l'animal indiquant un état de santé du membre respectif, (b) un score de vie naturelle de l'animal, indiquant la conformité d'un modèle de comportement du membre respectif avec un modèle de comportement naturel désiré, ou (c) un score d'affectivité/bonheur de l'animal, indiquant la conformité d'une mesure d'affectivité/bonheur du membre respectif avec une mesure d'affectivité/bonheur désirée, la mesure d'affectivité/bonheur étant déterminée sur la base d'un ou plusieurs des paramètres surveillés suivants, qui sont des paramètres d'affectivité/bonheur : (1) le niveau de respiration du membre donné, (2) le pourcentage du temps de rumination au cours d'une quatrième période de temps du membre donné, ou (3) le pourcentage du temps d'alimentation au cours d'une cinquième période de temps du membre donné ; et
dans lequel :
(iii) le score de santé est calculé en fonction des scores de santé des animaux calculés pour le premier groupe de membres donnés ; et
(iv) le score de bien-être est calculé en fonction de l'un au moins des éléments suivants : (a) le score de santé, (b) les scores de vie naturelle des animaux calculés pour le premier groupe de membres donnés, ou (c) les scores d'affectivité/bonheur des animaux calculés pour le premier groupe de membres donnés.

10. Système selon la revendication 3, ou procédé selon la revendication 9, dans lequel les paramètres surveillés incluent un ou plusieurs des paramètres comportementaux suivants : (a) un pourcentage de temps de mouvement dans une première période de temps du membre donné, (b) un pourcentage de temps d'alimentation dans une deuxième période de temps du membre donné ou (c) un pourcentage de temps de comportement social dans une troisième période de temps du membre donné ; et dans lequel le score de vie naturelle de l'animal pour le membre donné est déterminé sur la base des paramètres comportementaux.

11. Système selon la revendication 10, ou procédé selon la revendication 10, dans lequel le score de vie naturelle pour l'élément donné est déterminé sur la base de la cohérence de valeurs d'au moins certains des paramètres comportementaux dans le temps.

12. Système selon la revendication 3, ou procédé selon la revendication 9, dans lequel le score d'affectivité/bonheur pour le membre donné est déterminé sur la base de la cohérence de valeurs d'au moins certains des paramètres d'affectivité/bonheur dans le temps.

13. Système selon la revendication 2 ou 3, ou procédé selon l'une quelconque des revendications 8 ou 9, dans lequel le score de bien-être est calculé sur la base d'une variation entre les scores d'affectivité/bonheur du premier groupe de membres donnés.

14. Système selon la revendication 3, ou procédé selon la revendication 9, dans lequel les enregistrements incluent également un ou plusieurs paramètres environnementaux, indicatifs de l'état de l'environnement du membre respectif et dans lequel la détermination (a) du score de vie naturelle de l'animal du membre respectif ou (b) du score d'affectivité/bonheur de l'animal du membre respectif, est également basée sur les paramètres environnementaux.

15. Support de stockage non transitoire lisible par ordinateur sur lequel est incorporé un code de programme lisible par ordinateur, le code de programme lisible par ordinateur étant exécutable par au moins un circuit de traitement (730) d'un ordinateur pour mettre en œuvre un procédé d'estimation d'émissions de gaz à effet de serre dans un environnement logeant une population animale, le procédé comprenant :
l'obtention, par le circuit de traitement (730), d'au moins un sous-ensemble de deux enregistrements ou plus, chacun des enregistrements (i) étant associé à un membre respectif de membres de la population animale, et (ii) incluant un ou plusieurs paramètres surveillés du membre respectif tels que surveillés dans le temps par au moins l'un d'un groupe d'un ou plusieurs dispositifs de surveillance configurés pour surveiller des paramètres des membres de la population animale, le sous-ensemble étant associé à un premier groupe de membres donnés des membres ;
la détermination, par le circuit de traitement (730), d'un ou plusieurs paramètres affectant les émissions de gaz à effet de serre sur la base du sous-ensemble, au moins un paramètre affectant les émissions de gaz à effet de serre des paramètres affectant les émissions de gaz à effet de serre étant déterminé sur la base d'un ou de plusieurs des paramètres surveillés qui sont surveillés sur au moins deux membres du premier groupe de membres donnés ;
l'estimation, par le circuit de traitement (730), d'une quantité d'émissions de gaz à effet de serre dans l'environnement en utilisant les paramètres affectant les émissions de gaz à effet de serre en se basant au moins en partie sur une comparaison d'au moins un paramètre affectant les émissions de gaz à effet de serre avec au moins un niveau de référence déterminé à l'aide de mesures d'émissions de gaz à effet de serre acquises dans l'environnement abritant la population animale au moyen d'un équipement de mesure d'émissions de gaz à effet de serre ; et
la suggestion, par le circuit de traitement (730), d'une action en utilisant la quantité estimée d'émissions de gaz à effet de serre dans l'environnement.
